# EUROPEAN PATENT SPECIFICATION

(11) **EP 3 201 343 B1**
(45) Date of publication and mention of the grant of the patent: **23.03.2022**
(21) Application number: 15847855.2
(22) Date of filing: 29.09.2015
(51) Int. Cl.: C12N 15/85, C12N 15/00, A01K 67/00, C12N 15/873, C12N 15/90

(54) **HIGH EFFICIENCY, HIGH THROUGHPUT GENERATION OF GENETICALLY MODIFIED MAMMALS BY ELECTROPORATION**
HOCHEFFIZIENTE HOCHDURCHSATZERZEUGUNG VON GENETISCH MODIFIZIERTEN SÄUGETIEREN DURCH ELEKTROPORATION
GÉNÉRATION EXTRÊMEMENT EFFICACE ET À RENDEMENT ÉLEVÉ DE MAMMIFÈRES GÉNÉTIQUEMENT MODIFIÉS PAR ÉLECTROPORATION

(30) Priority: 29.09.2014 US 201462056687 P
(43) Date of publication of application: 09.08.2017
(73) Proprietor: The Jackson Laboratory, Bar Harbor,ME 04609 (US)
(72) Inventor: QIN, Wenning, Bar Harbor, ME 04609 (US); DION, Stephanie Lee, Bar Harbor, ME 04609 (US); KUTNY, Peter Michael, Ellsworth, ME 04605 (US); WANG, Haoyi, Bar Harbor, ME 04609 (US)
(74) Representative: Williams, Gareth Owen
(86) International application number: PCT/US2015/052928
(87) International publication number: WO 2016/054032

(56) References cited:
- WO-A1-01/32855
- WO-A1-2014/131833
- WO-A1-2014/131833
- CN-A- 102 329 819
- US-A1- 2011 023 156
- HUI PENG ET AL: "Efficient Delivery of DNA and Morpholinos into Mouse Preimplantation Embryos by Electroporation", PLOS ONE, vol. 7, no. 8, 21 August 2012 (2012-08-21) , page e43748, XP55331643, DOI: 10.1371/journal.pone.0043748

## Description

### BACKGROUND OF THE INVENTION

Delivery of biological or genetic material into mammalian zygote is usually the first step to modify the genome of a mammal, such as the creation of a genetically modified animal model. This is traditionally and presently accomplished by microinjection of the desired biological / genetic material into the zygote by using a glass needle.

Direct microinjection of DNA has been used to introduce Herpes Simplex virus (HSV) TK gene into cultured mammalian cells as early as in late 1980 (Capecchi, Cell, 22:479-488, November 1980). However, the efficiency of transformation was extremely low even when the small pBR 322/TK DNA was coinjected with SV40 DNA, *i.e.,* 15 transformants per 1,000 cells injected. Additionally, the experiment did not result in the production of a mature organism, much less one that could exhibit a phenotypic alteration.

About a month later, Gordon et al. (Proc. Natl. Acad. Sci. U.S.A., 77:7380-7384, December 1980) first reported that they had successfully microinj ected the TK gene (which was incorporated into a chimeric SV40 viral vector) into mice embryos, and had observed such TK genes in mice developed from the microinjected embryos. These mice, however, did not express the TK gene product. That is, in these experiments, no phenotypic alteration of the test animal was accomplished. The results reported by Gordon *et al.* seems to be consistent with an earlier study by Jaenisch et al. (Proc. Natl. Acad. Sci. U.S.A., 71:1250-1254, 1974) in which the SV40 virus had previously been shown to be functionally and genetically inactive when introduced into mouse embryos.

Subsequently, in June 1981, Wagner and Hoppe filed a U.S. utility application directed to the introduction of exogenous genetic material into embryos through microinjection, which application eventually leads to the issuance of U.S. Pat. No. 4,873,191, a broadly licensed foundation patent in the microinjection field that describes and broadly claims a microinjection method of obtaining a mammal having cells containing and capable of expressing exogenous genetic material.

Despite the numerous improvements since the early 1980s', microinjection remains technically demanding, labor intensive, and time consuming. Successful microinjection, to a great extent, depends on such subjective factors as technical proficiency, training, and experience of the operator. As it requires significant upfront investment in expensive microinjection setup and operators with years of training and experience, the capacity for generating genetically modified mouse models is often limiting even in the best and the most well-funded academic institutions of the world.

In addition, microinjection is inherently low in throughput, requiring manipulating the zygotes one at a time, thus limiting the size and scale of genome engineering experiments.

Electroporation, or electropermeabilization, is a process to significantly increase cell plasma membrane permeability and electrical conductivity, by using an externally applied electrical field. Electroporation has been used in molecular biology as a way of introducing some substances into a cell, such as loading it with a molecular probe, a drug that can change the cell's function, or a DNA molecule.

In molecular biology, the process of electroporation is often used for the transformation of bacteria, yeast, and plant protoplasts. In addition to the lipid membranes, bacteria also have cell walls which are different from the lipid membranes, and are made of peptidoglycan and its derivatives. However, the walls are naturally porous and only act as stiff shells that protect bacteria from severe environmental impacts. If bacteria and plasmids are mixed together, the plasmids can be transferred into the cell after electroporation. Several hundred volts across a distance of several millimeters are typically used in this process.

This procedure can also be used in tissue culture cells, such as cultured mammalian cells. As opposed to transformation in bacteria, the process of introducing foreign DNAs into eukaryotic cells is commonly known as transfection. Electroporation has been used for transfecting cells in suspension using electroporation cuvettes.

Despite the great and obvious need to overcome the many obstacles of microinjection described above, including low efficiency / throughput and technical difficulty, electroporation has not been seen as a viable alternative approach by those skilled in the art, and there has not been reported use of electroporation for introducing genetic materials into mammalian embryos which subsequently develop into genetically modified animals.

Specifically, electroporation has previously been mainly used in tissue culture cells and post-implantation embryos in the mouse (see Mellitzer et al., Mech. Dev., 118:57-63 (2002); Akamatsu et al., Proc. Natl. Acad. Sci. U.S.A., 96:9885-9890 (1999); Potter et al., Proc. Natl. Acad. Sci. U.S.A., 81:7161-7165 (1984); Osumi and Inoue, Methods, 24:35-42 (2001); Fukuchi-Shimogori and Grove, Science 294:1071-1074 (2001); and Tabata and Nakajima, Neuroscience, 103:865-872 (2001)).

In one study, Nemec et al. (Theriogenology 31:233, 1989) described attempts to introduce a neomycin resistant gene (pSVNEO) into mouse 1-cell embryos *(i.e.,* zygotes) through electroporation. However, the DNA comprising the neomycin gene was first injected into the perivitelline space between the plasma membrane and the zona pellucida of the zygotes in order to, according to the authors, decrease the distance between the exogenous DNA and the pronuclei upon exposure to the electrical currents, before the zygotes were subjected to electroporation. The authors reported that, in one experiment, Southern blot analysis revealed no incorporation of the neomycin gene in 55 pups produced from 226 embryos transferred to pseudopregnant ICR recipients. In a follow up experiment with high salt medium, designed to increase the total energy transferred during the pulses of electroporation, the authors again reported that preliminary data suggest that germ line transmission was not successful.

More recently, electroporation has been successfully used to deliver dsRNA, siRNA, DNA vectors encoding the same, and morpholinos into mouse preimplantation embryos (Grabarek et al., Genesis, 32:269-276 (2002); Wang et al., Dev. Biol., 318:112-125 (2008); and Peng et al., PLoS ONE, 7(8):e43748. doi:10.1371/journal.pone.0043748 (2012)). However, these reagents work in the cytosol, not in the nucleus, as is required to bring about genetic modification that can be transmitted through germline. More specifically, these reagents work by inhibiting expression of their respective target genes, either by degrading the mRNA of the target gene, or by blocking the translation of the mRNA, or both.

US 2011/023156 describes a genetically modified feline or cell comprising at least one chromosomal sequence edited using a zinc finger nuclease-mediated editing process. CN 102 329 819 describes electroporation of mammalian embryos with siRNA. WO2014/131833 relates to a method of producing a non-human, mammalian oocyte carrying a modified target sequence in its genome, using a CRISPR-Cas9 system.

Thus, despite the long-felt need to overcome the many shortcomings of microinjection (such as low efficiency) for use in introducing exogenous biological or genetic material into early stage embryos to generate genetically modified animals through germline transmission, the prevailing art appears to teach away from a seemingly simpler technique such as electroporation.

### SUMMARY OF THE INVENTION

The present invention provides a method of generating a genetically modified mouse, as recited in claim 1. Further features of the invention are described in the dependent claims.

Described herein is a method of generating a genetically modified mouse, the method comprising introducing via electroporation a material into a preimplantation stage embryo of a mouse to modify the genome of the mouse.

In certain embodiments, the preimplantation stage embryo is a 1-cell embryo *(i.e.,* a zygote), a 2-cell embryo, a 4-cell embryo, an early morula, or a late morula.

The method further comprises allowing the (electroporated) preimplantation stage embryo to develop into a live-born genetically modified mouse.

The material comprises a polynucleotide comprising a coding sequence for a Type-II Cas9 protein, and a polynucleotide comprising a Clustered Regularly Interspaced Short Palindromic Repeats (CRISPR)-CRISPR associated (Cas) (CRISPR-Cas) system guide RNA that hybridizes to a target sequence in the genome of the mouse.

In certain embodiments, the concentration ratio for the coding sequence for the Type-II Cas9 protein and the CRISPR-Cas system guide RNA is at least about 1:1, at least about 1.5:1, at least about 2:1, at least about 2.5:1, at least about 3:1 or higher.

In certain embodiments, the concentration of the coding sequence for the Type-II Cas9 protein is at least 300 ng/µL, at least 400 ng/µL, at least 500 ng/µL, at least 600 ng/µL, at least 800 ng/µL, or at least 1000 ng/µL or higher.

In certain embodiments, the material further comprises a donor polynucleotide, wherein the donor polynucleotide is a linear or circular double- or single-stranded DNA molecule.

The preimplantation embryo, after electroporation, is transplanted into a pseudopregnant host mouse capable of bearing the embryo to term.

The preimplantation embryo is removed from a medium in which electroporation is carried out prior to being transplanted into the pseudopregnant host mouse.

In certain embodiments, about 2, 3, 5, 10, 20, 30, 40, 50, 100, 125, 150, 200, 250, 300 or more preimplantation stage embryos are simultaneously electroporated.

In certain embodiments, at least about 50%, 60%, 70%, 80%, 85%, 90%, 95% or more of the electroporated preimplantation stage embryos develop into live-born transgenic mice.

In certain embodiments, the gamete or the preimplantation stage embryo is pre-treated to weaken Zona Pellucida prior to electroporation.

In certain embodiments, the preimplantation stage embryo is pre-treated in Acidic Tyrode's solution (AT) or equivalent.

In certain embodiments, electroporation is carried out in a 1-mm electroporation cuvette, using the settings of: 20-40 volts *(e.g.,* 25, 26, or 27 volts), pulse duration 1-2 ms *(e.g.,* 1.5 ms), 1-3 pulses, pulse interval 100-1000 ms *(e.g.,* about 125-130 ms).

It should be understood that any embodiments described herein above and below are contemplated to be able to combine with any other embodiments of the invention, including those specific embodiments described only in the examples.

### BRIEF DESCRIPTION OF THE DRAWINGS

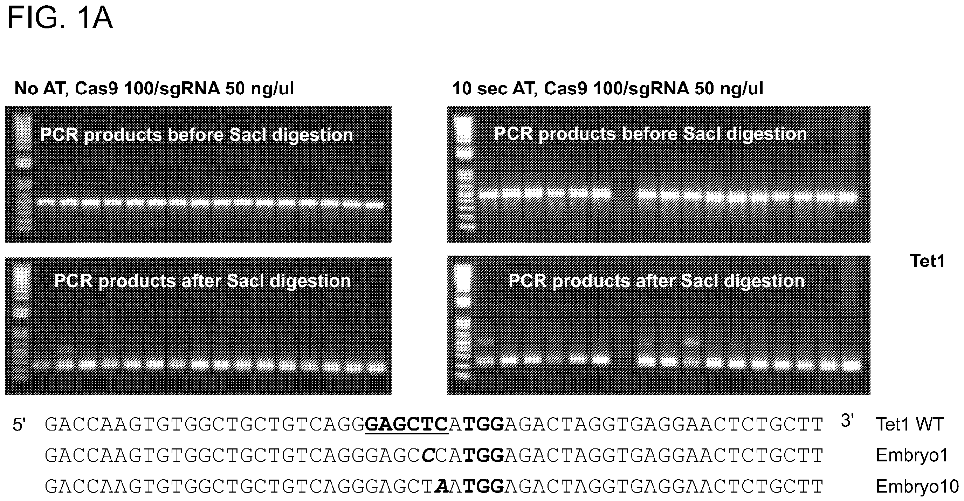

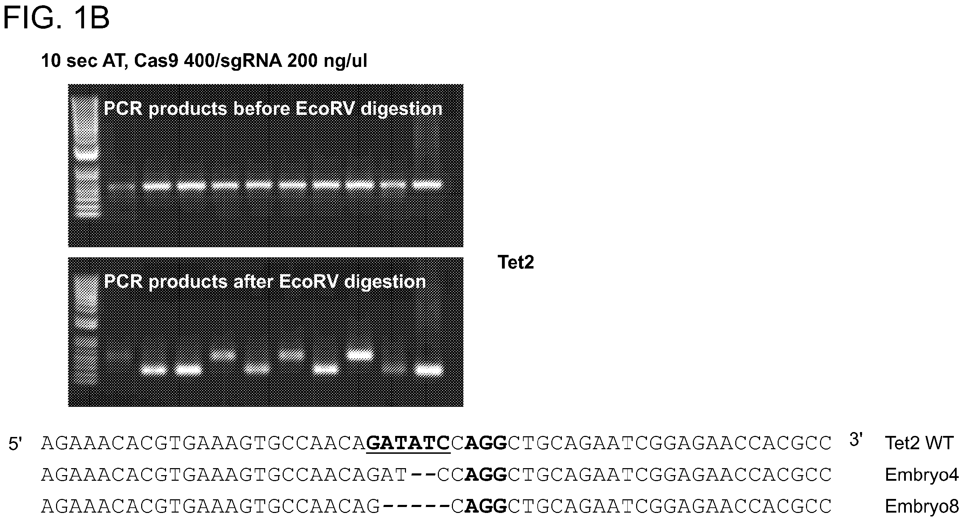

FIGs. 1A and 1B show CRISPR-mediated gene disruption in mouse embryos using the subject method. Specifically, FIG. 1A shows genotyping of embryos targeted at the Tetl locus. RFLP analysis is shown in the top panel and sequencing results are shown in the bottom panel. The restriction site at the target region, used for RFLP analysis, is bold and underlined. The protospacer-adjacent motif (PAM) sequence is colored in red. Mutant alleles from two embryos are shown, and the mutated bases are colored in blue. FIG. 1B shows genotyping of embryos targeted at the Tet2 locus.

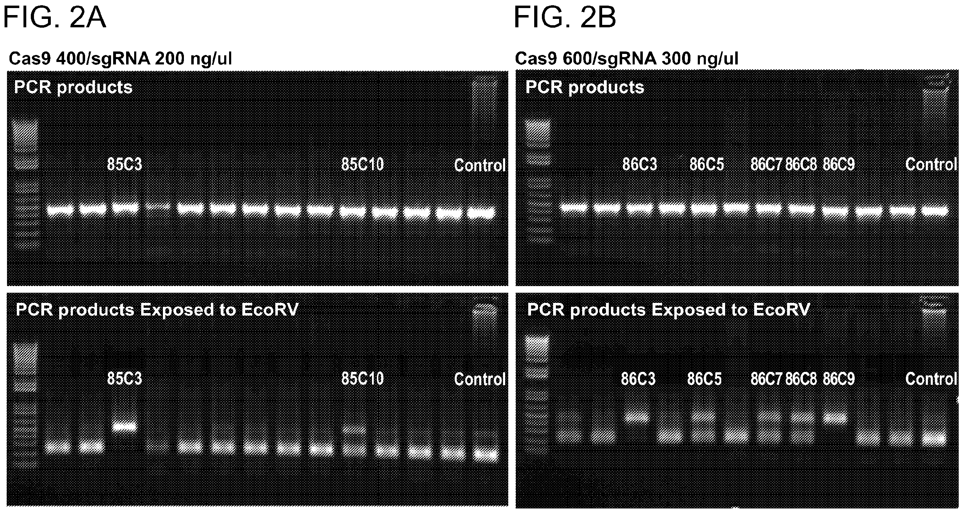

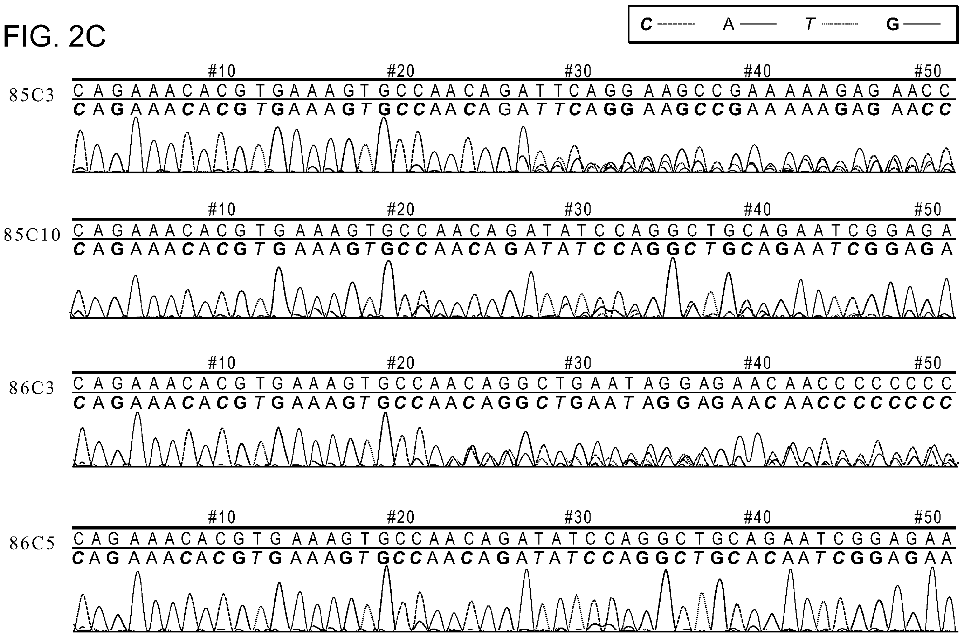

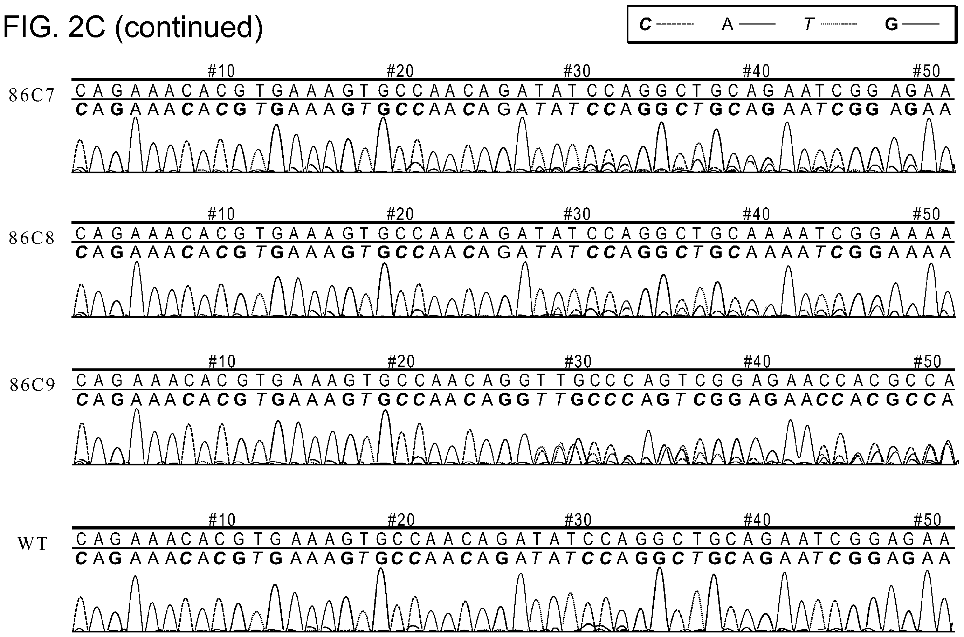

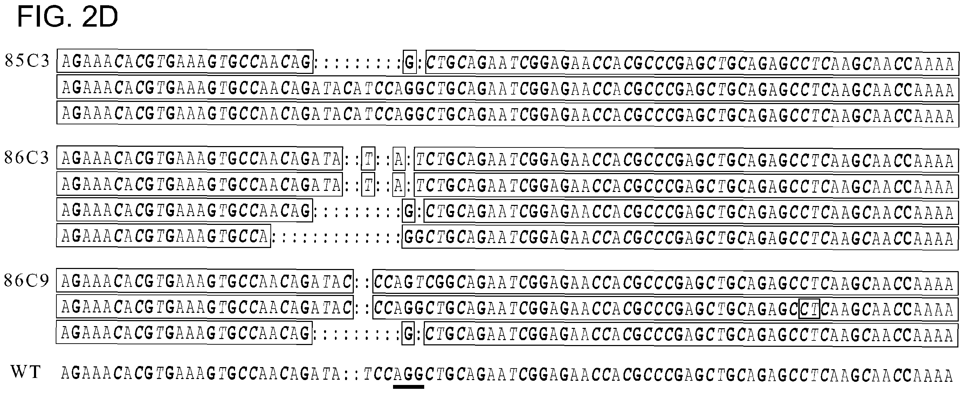

FIGs. 2A-2D show CRISPR-mediated gene disruption in live mice using the subject method. Specifically, in FIG. 2A: Cas9 mRNA and sgRNA (single-guide RNA) targeting the Tet2 locus and used at 400 ng/µL and 200 ng/µL, respectively, were delivered to the mouse embryo by electroporation. Electroporated embryos were then transferred to pseudopregnant female mice. Tail biopsy samples were taken from new born mice and genotyped using the RFLP method with EcoRV enzyme. PCR products from mice 85C3 is resistant and 85C10 is partially resistant to EcoRV digestion. In FIG. 2B: Cas9 mRNA and sgRNA targeting the Tet2 locus was used at 600 ng/µL and 300 ng/µL, respectively. PCR products from mice 86C3 and 86C9 were resistant, and those from mice 86C5, 86C7 and 86C8 were partially resistant to EcoRV digestion. In FIG. 2C: PCR products from mouse 85C3, 85C10, 86C3, 86C5, 86C7, 86C8, and 86C9 were processed by Sanger sequencing and the chromatogram was shown as compared to that from a wild type sample. FIG. 2D: PCR products from mouse 85C3, 86C3 and 86C9 were cloned into pCR2.1-Topo vector, and individual clones were sequenced. INDEL mutations were readily detected among alleles from all 3 founder mice.

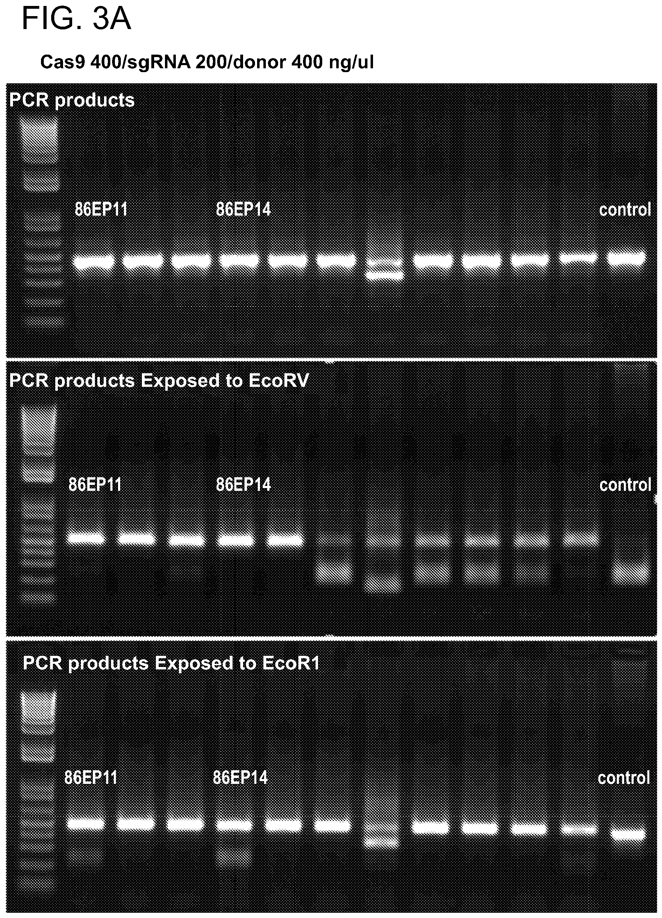

FIG. 3A shows results of genotyping of 11 founder mice from Experiment 15. The top panel shows PCR products without RFLP analysis. The middle panel shows RFLP analysis using EcoRV. The bottom panel shows RFLP analysis using EcoRI.

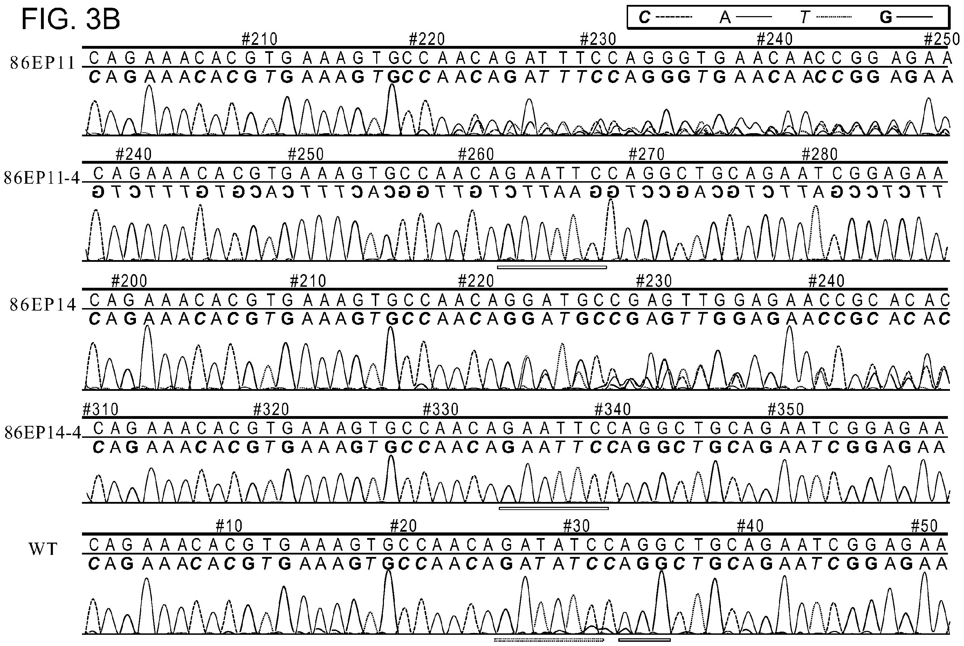

FIG. 3B shows Sanger sequencing results of selected founder mice and control mouse. Changed sequences (through CRISPR/Cas-mediated HDR) in relation to the wildetype sequence in the control mouse have been underlined.

### DETAILED DESCRIPTION OF THE INVENTION

### 1. Overview

To overcome the inherent limitations associated with microinjection in the context of generating genetically modified animals (e.g., transgenic or other genetically modified mammals), Applicant has developed electroporation-based methodology to deliver biological / genetic materials into preimplantation mouse embryos including zygotes, under specific conditions that renders the method amenable to high efficiency, high throughput generation of genetically modified animals with high survival rate. The resulting genetic modification can be readily transmitted through germline.

Specifically, in an exemplary embodiment, the methods described herein were used to deliver CRISPR/Cas system to mouse embryos (e.g., zygotes), thus effecting targeted genetic modification in the genome of the resulting genetically modified / transgenic mice. The methods described herein, including embodiments or protocols optimized herein, can be carried out using commercially available electroporator, while requiring minimal training or prior experience, and thus are able to process a large number of embryos and multiple gene targeting projects in parallel in a single step. Using the methods described herein, any of a variety of biological, genetic, or other materials, including but not limited to CRISPR/Cas, TALEN, and ZFN, or combination thereof, *etc.,* can be delivered to early stage mouse embryos (e.g., preimplantation mouse embryos including zygotes). Thus the technology described herein enables animal genome engineering with unprecedented ease and scale.

The invention described herein is partly based on the discovery that electroporation can be used to deliver relatively high concentrations (e.g., previously thought to be toxic level) of biological / genetic material into preimplantation embryos including zygotes, which high concentration may be required to achieve desired end results in the nucleus of the electroporated early stage embryos, such as genetic modification of the genome of the embryos, which modification can be passed on via germline transmission.

Specifically, based on prior microinjection experiments, in which relatively low concentrations of Cas9 mRNA (50 or 100 ng/µL) and sgRNA (25 or 50 ng/µL) were successfully used in a pronuclear or cytoplasmic microinjection experiments, Applicant attempted to electroporate zygotes using reported gene-encoding plasmids (see Example 1). Surprisingly, no fluorescence was observed in 3.5-day embryos developed from the electroporated zygotes. Similar experiments using other concentrations of DNA, with pretreatment of zona pellucida to weaken it; or with other reporters or fluorescein-labeled oligonucleotides, also failed. See Experiments 2-6 mentioned in Example 1.

It is widely believed that the reagents used for the electroporation experiment, including the Cas9 mRNA and the guide RNA, may be toxic to the embryos when used at a higher concentration.

Applicant has demonstrated herein that early stage embryos such as zygotes can be electroporated using relatively high concentrations of biological materials (e.g., polynucleotides comprising CRISPR/Cas mRNA and guide RNA) to effect the desired genetic modifications in embryos or animals developed from the electroporated embryos. See Example 2.

The invention described herein is further partly based on the discovery that electroporated preimplantation embryos including zygotes usually exhibit retarded or arrested development, or otherwise do not survive to live-born animal, unless the electroporated preimplantation embryos including zygotes are released into a physiological solution or medium. For example, the solution or medium, among other things, is isotonic. In addition, by releasing the electroporated preimplantation embryos including zygotes into the physiological solution or medium, the usual electroporation medium is removed (e.g., buffers used to dissolve polynucleotide such as TE), thus further lessening the chance that any harmful substances therein could retard subsequent embryonic development.

For example, when the buffer or medium used for electroporation is not a physiological solution or medium, such as containing excessive (e.g., more than 40% or 50%) buffer used to dissolve materials to be electroporated, it may be helpful to remove such buffer soon after electroporation.

In certain embodiments, when TE or other similar buffer commonly used to dissolve nucleic acid reagents is present in significant amount (e.g., >40% or 50%) in the electroporation medium, several means may be used to mitigate any potential harmful effects. For instance, in one embodiment, the time embryos are bathed in TE buffer may be minimized by immediately washing the electroporated embryos in a physiological solution after electroporation. In other embodiment, the nucleic acid reagents (such as the CRISPR/Cas reagents) dissolved in TE buffer can be mixed in proper proportion with a physiological solution such that overall osmolarity is brought closer to physiological. In yet another embodiment, the nucleic acid reagents (e.g., CRISPR/Cas reagents) can be reconstituted directly in a physiological solution to avoid or minimize any complications. That is, the preimplantation embryos including zygotes may be electroporated in a physiological solution or medium, e.g., one that is isotonic. The physiological solution or medium may be an artificially prepared solution similar to blood plasma in salt composition and osmotic pressure.

Although electroporation can indeed introduce biological material into zygotes under the conditions described herein, survival of the zygotes in subsequent development is required to create live-born animals. Initial failures in culturing electroporated zygotes appear to suggest that zygotes may not survive the electroporation process and continue to develop into live-born animal, regardless of whether the desired genetic modification occurs in the electroporated zygotes. The realization that AT treatment could damage the zygotes excessively to retard embryonic development, and that the reagents delivered, such as the Cas9 mRNA and the guide RNA, could be toxic to the zygotes and their subsequent development, further teaches away from using electroporation as a viable means to permanently introduce germline transmittable genetic modifications in the animal.

Applicant has demonstrated herein that survival rate of the electroporated zygotes, *e.g.,* as measured by successful development into blastocyst stage embryos, can be dramatically improved to surprisingly high levels *(e.g.,* close to or even reaching 100%) using the methods of the invention.

In embodiments of the invention, the material is introduced into an early stage embryo, such as a preimplantation stage embryo. The preimplantation stage embryo may be a 1-cell embryo *(i.e.,* a zygote), a 2-cell embryo, a 4-cell embryo, an 8-cell embryo, an early morula, or a late morula. Since embryonic development may sometimes involve asymmetric division, it is possible that, at least transiently, the early state embryo is a 3-, 5-, 6-, 7-cell embryos, or other embryos that are not the result of symmetric embryonic division. The methods described herein also apply to such early stage embryos. In certain embodiments, the method further comprises allowing the (electroporated) preimplantation stage embryo to develop into a live-born genetically modified animal (e.g., mammal). For example, the preimplantation embryo, after electroporation, may be transplanted into a pseudopregnant host animal *(e.g.,* mammal), preferably of the same species or strain, which animal *(e.g.,* mammal) is capable of bearing the embryo to term.

In certain embodiments, the preimplantation embryo is first removed from a medium in which electroporation is carried out, prior to being transplanted into the pseudopregnant host animal (e.g., mammal). For example, the electroporated preimplantation embryos can be washed one or more times in a medium (e.g., an isotonic physiological solution or medium) suitable or compatible for embryonic development. Such wash also removes any substances in the electroporation medium or buffer that may be harmful or otherwise not conducive for further embryonic development *in vitro* or *in vivo.*

Suitable medium for this purpose includes, but are not limited to M2-medium or OPTI-MEM^{®} or any other physiological solution (with the appropriate ionic strength and pH). In certain embodiments, the medium or buffer used for washing is pre-warmed to an optimal temperature *(e.g.,* about 37°C) for embryonic development of the animal *(e.g.,* mammal), to reduce or minimize any potential temperature shock.

In certain embodiments, the electroporation medium is isotonic.

In certain embodiments, the electroporated preimplantation embryos are washed to remove one or more buffers used to dissolve a polynucleotide, such as TE buffer, or an equivalent buffer that contains metal cheaters such as EDTA.

In certain embodiments, the electroporated preimplantation embryos are washed to remove basal or reduced-serum medium, such as OPTI-MEM^{®} medium (INVITROGEN, Carlsbad, CA), siPORT^{™} (AM8990 or AM8990G; Ambion, Austin, TX), or mixture or equivalent thereof, that may be used as electroporation medium.

Numerous reagents or materials may be introduced into the preimplantation embryos using the methods described herein, in order to bring about genetic modification that can be transmitted through germline.

In certain embodiments, the material may comprise a polynucleotide, a polypeptide, or both. In other embodiments, additional materials, e.g., those chemical in nature or synthetic such that they promote or inhibit the NHEJ, HDR or HR process, may also be introduced via electroporation. For example, certain chemical reagents may be included to facilitate or enhance the function of the CRISPR/Cas system (see below).

In certain embodiments, the polynucleotide comprises a coding sequence for a Type-II Cas9 protein. In an exemplary embodiment, the coding sequence for the Cas9 protein is an mRNA, such as a codon-optimized mRNA suitable for expression in an eukaryotic cell.

In certain embodiments, the polynucleotide may comprise a Clustered Regularly Interspaced Short Palindromic Repeats (CRISPR)-CRISPR associated (Cas) (CRISPR-Cas) system guide RNA that is capable of hybridization to a target sequence in the genome of the mammal. The guide RNA only needs to be sufficiently similar in sequence to the target sequence such that they can hybridize under intracellular physiological conditions. In certain embodiments, however, the guide RNA is fully complementary (or 100% identical) to the target sequence.

In certain embodiments, the guide RNA may be a guide sequence fused to a trans-activating cr (tracr) sequence.

In certain embodiments, concentration ratio for the coding sequence for the Type-II Cas9 protein and the CRISPR-Cas system guide RNA may vary. For example, suitable ratio may be at least about 1:1, at least about 1.5:1, at least about 2:1, at least about 2.5:1, at least about 3:1 or higher.

In certain embodiments, the concentration of the coding sequence for the Type-II Cas9 protein is at least 200 ng/µL, at least 300 ng/µL, at least 400 ng/µL, at least 500 ng/µL, at least 600 ng/µL, at least 800 ng/µL, or at least 1000 ng/µL or higher.

In certain embodiments, the material further comprises a donor polynucleotide (e.g., to facilitate CRISPR-mediated NHEJ, HDR or HR). For example, the donor polynucleotide may be a linear or circular double- or single-stranded DNA molecule. Such donor polynucleotides may be included to facilitate site-specific modification of or around a target sequence which may be the target of the introduced CRISPR-Cas system.

The polynucleotide may comprise a DNA vector, or an RNA *(e.g.,* an mRNA, and/or a CRISPR guide RNA).

The present disclosure has application in the genetic modification of multicellular eukaryotic animals which undergo syngamy, *i.e.,* sexual reproduction by the union of gamete cells. Examples of such animals include amphibians, reptiles, birds, mammals, bony fishes, cartilaginous fishes, cyclostomes, arthropods, insects, mollusks, and thallophytes. Exemplary animals include mammals, birds, and fishes.

In certain embodiments as claimed herein, the animal is a mouse.

The methods described herein can be used to introduce numerous germline transmissible modifications to the genome of the animal species involved, including but not limited to: small insertions or deletions (e.g., those of one or more base pairs), insertions of a heterologous DNA fragment, deletions of an endogenous DNA fragment, inversion or translocation of an endogenous DNA fragment, or a combination thereof. Likewise, numerous different mechanisms may be involved in bringing about the modifications, including but not limited to non-homologous end joining (NHEJ), homology-directed repair (HDR), or homologous recombination (HR).

Homology directed repair (HDR) is a mechanism in cells to repair double strand DNA lesions. This repair mechanism can only be used by the cell when there is a homologue piece of DNA present in the nucleus, mostly in G2 and S phase of the cell cycle.

When the homologue DNA piece is absent, another process called non-homologous end joining (NHEJ) can take place. Non-homologous end joining (NHEJ) is another pathway that repairs double-strand breaks in DNA. NHEJ is referred to as "non-homologous" because the break ends are directly ligated without the need for a homologous template, in contrast to homologous recombination, which requires a homologous sequence to guide repair. NHEJ typically utilizes short homologous DNA sequences (e.g., microhomologies) to guide repair. These microhomologies are often present in single-stranded overhangs on the ends of double-strand breaks. When the overhangs are perfectly compatible, NHEJ usually repairs the break accurately. Imprecise repair leading to loss of nucleotides can also occur, but is much more common when the overhangs are not compatible. Inappropriate NHEJ can lead to translocations and telomere fusion. NHEJ is evolutionarily conserved throughout all kingdoms of life, and is the predominant double-strand break repair pathway in mammalian cells.

When the NHEJ pathway is inactivated, double-strand breaks can be repaired by the more error-prone pathway known as microhomology-mediated end joining (MMEJ). In this pathway, end resection reveals short microhomologies on either side of the break, which are then aligned to guide repair. This contrasts with classical NHEJ, which typically uses microhomologies already exposed in single-stranded overhangs on the DSB ends. Repair by MMEJ therefore leads to deletion of the DNA sequence between the microhomologies.

Homologous recombination is a type of genetic recombination in which nucleotide sequences are exchanged between two similar or identical molecules of homologous DNA. It is most widely used by cells to accurately repair harmful breaks that occur on both strands of DNA, known as double-strand breaks (DSBs). Homologous recombination is also the process that produces new combinations of DNA sequences during meiosis, the process by which eukaryotes make gamete cells, like sperm and egg cells in animals. Homologous recombination is conserved across all three domains of life as well as in viruses.

The methods described herein are suitable for preimplantation embryo, and are also suitable for multiplexing and simultaneous use for about 2, 3, 5, 10, 20, 30, 40, 50, 100, 125, 150, 200, 250, 300 or more preimplantation stage embryos, e.g., all can be simultaneously electroporated, either in the same electroporation cuvette, or multiple similar or identical ones, under the same or different conditions.

The methods described herein are superior to microinjection in many respects. Compared to microinjection, the subject method is not only much more efficient and much less technically demanding and amenable for standardization, the survival rate of embryos and the rate of germline transmission are both much higher. For example, microinjection can typically achieve a survival rate of about 20-25% for injected mice embryos (e.g., about 20-25% of the injected mice embryos survive to yield live-born mice pups). In comparison, at least about 50%, 60%, 70%, 80%, 85%, 90%, 95% or more of the electroporated preimplantation stage embryos develop into blastocyst stage embryos, preferably at a rate comparable to a matching control, *e.g.,* in about 3.5 days post electroporation for a zygote, or develop into live-born transgenic mammals. For example, the matching control can be sham electroporated embryos, which may be electroporated by empty vector, scrambled mRNA and/or guide sequence for CRISPR/Cas, or buffer only.

In certain embodiments, at least about 70%, 75%, 80%, 85%, 90% or more of the electroporated preimplantation stage embryos develop into live-born transgenic mammals.

In certain embodiments, targeting efficiency of the host genome, as measured by the percentage of live-born mammals that contain the genetic modification as designed, is at least about 80%, 85%, 90%, 95%, 97%, 99%, or close to 100%.

While not wishing to be bound by any particular theory, the high targeting efficiency of the subject method may be owing to the fact that the embryos are submerged in a homogenous CRISPR/Cas solution and upon electroporation, have similar exposure to the CRISPR/Cas reagents. This is in direct contrast to a corresponding microinjection experiment, in which the level of technical competency and factors surrounding a particular experiment may all contribute to relatively large variations in targeting efficiency.

In certain embodiments, the the preimplantation stage embryo is pre-treated to weaken Zona Pellucida prior to electroporation. For example, the the preimplantation stage embryo can be pre-treated in Acidic Tyrode's solution (AT) for this purpose.

Tyrode's solution was invented by American pharmacologist Maurice Vejux Tyrode, and is a modification of Ringer-Locke's solution. It is roughly isotonic with interstitial fluid, and is mainly used in physiological experiments and tissue culture. It contains magnesium, a sugar (usually glucose) as an energy source, and uses bicarbonate and/or HEPES as a buffer. Some variations also include phosphate and sulfate ions. It is typically gassed with 95% oxygen 5% carbon dioxide when used for cell culture applications and physiology experiments.

Acidic Tyrode's solution can typically be prepared by dissolving in 100 mL of water the following at room temperature and adjusting to pH 2.5 with Analar HCl (BDH): NaCl, 0.8 g; KCl, 0.02 g; CaCl₂•2H₂O, 0.024 g; MgCl₂•6H₂O, 0.01 g; Glucose, 0.1 g; Polyvinylpyrrolidone (PVP), 0.4 g. The PVP is added to increase viscosity and reduce embryo stickiness. The solution can be filter-sterilized and stored in aliquots at -20°C. AT is commercially available from venders such as Sigma-Aldrich (e.g., SKU T1788 for 100 mL packages).

In certain embodiments, the preimplantation embryos are pre-treated in AT for about 5-30 seconds, about 5-20 second, or about 5-10 seconds. Longer treatment time may also be used if the AT is diluted. Suitable AT treatment time can be empirically determined by treating preimplantation embryos over different time periods, followed by measuring viability, the percentages of damaged or apoptotic embryos post treatment, or survival rate of embryos during subsequent development.

Although slightly different electroporation conditions may be used, in certain embodiments, electroporation in the present disclosure is carried out in a 1-mm electroporation cuvette, using the settings of: 20-30 volts *(e.g.,* 25, 26, or 27 volts), pulse duration 1-2 ms *(e.g.,* 1.5 ms), 1-3 pulses, pulse interval 100-1000 ms *(e.g.,* about 125-130 ms). In certain embodiments, 2-mm cuvette may also be used with similar parameters with appropriate adjustments.

In certain embodiments, the electroporated embryos may be used immediately after electroporation, e.g., transplanting into a pseudopregnant female. In other embodiments, the preimplantation stage embryo, after electroporation, is frozen for storage and then thawed for the next step, e.g., transplanting into a pseudopregnant female. In yet another embodiment, the electroporated embryos can first be allowed to develop into a later stage embryo, such as an early or late morula, or a blastocyst stage embryo, before cryopreservation.

Cryopreservation can be done using standard techniques, such as vitrification or slow programmable freezing (SPF).

In certain embodiments, the preimplantation embryos, zygotes used for electroporation are obtained by *in vitro* fertilization (IVF), recovered from cryopreservation.

Another aspect of the present disclosure provides a genetically modified animal (e.g., mammal) generated by any one of the methods described herein.

With the invention generally described above, the sections below provide additional details about specific aspects of the invention that should be read in combination and in the context of the general description.

Any one of the specific embodiments described herein is contemplated to be combinable with any one or more other embodiments of the invention, including those described only in the Example section.

### 2. Electroporation and Electroporators

Electroporation is a dynamic phenomenon that depends on the local transmembrane voltage at each point on the cell membrane. For a given pulse duration and shape, a specific transmembrane voltage threshold exists for the manifestation of the electroporation phenomenon (e.g., from 0.5 V to 1 V). This leads to the definition of an electric field magnitude threshold for electroporation (Eₜₕ). Only cells within areas where E≧Eₜₕ are electroporated. If a second threshold (Eᵢᵣ) is reached or surpassed, electroporation will compromise the viability of the cells, resulting in irreversible electroporation (IRE).

Electroporation allows cellular introduction of large highly charged molecules, such as DNA, RNA or protein, which may not passively diffuse across the hydrophobic bilayer core. While not wishing to be bound by any particular theory, the mechanism of electroporation is believed to involve the creation of nm-scale water-filled holes in the cell membrane. It is believed that, during electroporation, the lipid molecules in the membrane are not chemically altered, but simply shift position, opening up a pore which acts as the conductive pathway through the bilayer as it is filled with water.

Electroporation is usually a multi-step process with several distinct phases. First, a short electrical pulse must be applied. Typical parameters would be 300-400 mV for < 1 ms across the membrane. The voltages used in cell experiments are typically much larger because they are being applied across large distances to the bulk solution so the resulting field across the actual membrane is only a small fraction of the applied bias. Upon application of this potential, the membrane charges like a capacitor through the migration of ions from the surrounding solution. Once the critical field is achieved there is a rapid localized rearrangement in lipid morphology. The resulting structure is believed to be a "prepore" since it is not electrically conductive but leads rapidly to the creation of a conductive pore. Evidence for the existence of such pre-pores comes mostly from the "flickering" of pores, which suggests a transition between conductive and insulating states. It has been suggested that these pre-pores are small (~3 Å) hydrophobic defects. If this theory is correct, then the transition to a conductive state could be explained by a rearrangement at the pore edge, in which the lipid heads fold over to create a hydrophilic interface. Finally, these conductive pores can either heal, resealing the bilayer or expand, eventually rupturing it. The resultant fate depends on whether the critical defect size was exceeded, which in turn depends on the applied field, local mechanical stress, and bilayer edge energy.

According to the present disclosure, electroporation of the preimplantation embryos can be done with commercially available electroporators, appliances that create an electromagnetic field in the cell solution. Exemplary but non-limiting commercial models of electroporators include: ECM^{™} 830 Square Wave Electroporator, or 2001 Electro Cell Manipulator (ECM 2001) from BTX (San Diego, CA).

In a typical setting, preimplantation embryo suspension is pipetted into a glass or plastic cuvette, which has two aluminum electrodes on its sides. Prior to electroporation, the preimplantation embryos are gently mixed with the (biological) materials (e.g., DNA, RNA, or protein, or mixture thereof) to be introduced into the preimplantation embryos. The mixture can be made either before its pipetting into the cuvette *(e.g.,* a 1-mm or 2-mm width cuvette), or made in the cuvette. Typically, a suspension mixture of around 20 µL total volume is used, although 10-200 µL, 20-100 µL, 25-75 µL total volume may also be used. Then the voltage and capacitance are set (see below), and the cuvette is inserted into the electroporator. Immediately after electroporation, a given amount of *(e.g.,* equal volume as the entire cuvette content, about 20-200 µL, or about 100 µL) of a liquid medium optimal for embryo survival is added to the cuvette to wash out the electroporated mixture, and the entire content is collected in an appropriate container (e.g., tissue culture dish).

Survival of the electroporated preimplantation embryos and the development thereof into culture or live-born animal to some extent depends on careful wash and removal of the electroporation medium if it is not a physiological solution. The physiological solution not only provides proper isotonic environment for embryo development, but may also remove any potentially harmful substances that may not be conductive for embryo development /survival.

The preimplantation embryos, preferably after wash, are transferred to pseudopregnant females or incubated at optimal temperature and/or atmospheric conditions *(e.g.,* at 37°C, 5% CO₂ or 5% O₂ / 5% CO₂ / Nitrogen) for a required period of time before the developing embryos, such as the 3.5-day blastocyst stage embryos, are transferred to surrogate mother / pseudopregnant female to develop to full term and be born as live animal.

A typical electroporation condition, as can be used in the ECM 830 or ECM 2001 model, may include carrying out electroporation in a 1-mm electroporation cuvette, using the settings of: 20-30 volts *(e.g.,* 25, 26, or 27 volts), pulse duration 1-2 ms *(e.g.,* 1.5 ms), 1-3 pulses, pulse interval 100-1000 ms (e.g., about 125-130 ms). However, it should be understood that the conditions disclosed herein are for illustrative purpose and are not limiting. One of skill in the art can readily adjust the parameters based on, for example, the size and amount of the molecules to be introduced, the type of embryos, *etc.*

Electroporation can be done in commercially available bench top electroporators, such as the ECM830 or ECM 2001 model from BTX, and the NUCLEOFECTOR^{®} Devices (Lonza Group Ltd.). Some units offer the possibility of electroporating multiple samples at the same time with special electrode assemblies that fit into multi-well cell culture dishes. Bench top electroporators can also be set to different operating parameters, allowing operators to explore or optimize specific parameters, such as field strengths.

### 3. Mammals

In certain embodiments, the mammal is a mouse, such as an inbred mouse.

Exemplary inbred mouse strains include, without limitation, inbred mouse lines C3H, *e.g.,* CBA, DBA, FVB, NOD, BALB/c, 129, C57BL, and C57BL mice including C57BL/6J, C57BL/6NTac, C57BL/6NCrl, C57BL/10, *etc.*

Other inbred mouse strains, including recombinant inbred strains, include (without limitation): 129S1/SvImJ, 129T2/SvEmsJ, 129X1/SvJ, 129P3/J, A/J, AKR/J, BALB/cBy, BALB/cByJ, BALB/c BALB/cJ, C3H/HeJ, C3H/HeOuJ, C3HeB/FeJ, C57BL/10J, C58, CBA/CaHN-Btkxid/J, CBA/J, DBA/1J, DBA/1LacJ, DBA/2J, FVB/NJ, MRL/MpJ, NOD/LtJ, SJL/J, SWR/J, NOR/LtJ, NZB/BlNJ, NZW/LacJ, RBF/DnJ, 129S6/SvEvTac, AJTAC, BALB/cAnNTac, BALB/cJBomTac, BALB/cABomTac, C57BL/6NTac, C57BL/6JBomTac, C57BL/10SgAiTac, C3H/HeNTac, CBA/JBomTac, DBA/1JBomTac, DBA/2NTac, DBA/2JBomTac, FVB/NTac, NOD/MrkTac, NZM/AegTac, SJL/JcrNTac, BALB/cAnNCrlBR, C3H/HeNCrlBR, C57BL/6NCrlBR, DBA/2NCrlBR, FVB/NCrlBR, C.B-17/IcrCrlBR, 129/SvPasIcoCrlBR, SJL/JorlIcoCrlBR, A/JolaHsd, BALB/cAnNHsd, C3H/HeNHsd, C57BL/10ScNHsd, C57BL/6NHsd, CBA/JCrHsd, DBA/2NHsd, FVB/NHsd, SAMP1/KaHsd, SAMP6/TaHsd, SAMP8/TaHsd, SAMP10/TaHsd, SJL/JCrHsd, AKR/OlaHsd, BiozziABH/RijHsd, C57BL/6JOlaHsd, FVB/NhanHsd, MRL/MpOlaHsd, NZB/OlaHsd, NZW/OlaHsd, SWR/OlaHsd, 129P2/OlaHsd, and 129S2/SvHsd, B6.129P2-Apoetm1Unc/J, NOD.CB17-Prkdcscid/J, 129S1/SvImJ, 129X1/SvJ, B10.A-H2a H2-T18a/SgSnJ, B10.D2-Hc0 H2d H2-T18c/oSnJ, B10.D2-Hc1 H2d H2-T18c/nSnJ, B10.RIII-H2r H2-T18b/(71NS)SnJ, B6(C)-H2-Ab1bm12/KhEgJ, B6.129P2-Il10tm1Cgn/J, B6.129P2-Nos2tm1Lau/J, B6.129P2-Nos3tm1Unc/J, B6.129S2-Cd8atm1Mak/J, B6.129S2-Igh-6tm1Cgn/J, B6.129S7-Ifngtm1Ts/J, B6.129S7-Rag1tm1Mom/J, B6.CB17-Prkdcscid/SzJ, B6.MRL-Faslpr/J, B6.V-Lepob/J, BKS.Cg-m +/+ Leprdb/J, C3HeB/FeJ, C57BL/10J C57BL/10SnJ, C57BL/6-Tg(APOA1)1Rub/J, C57BL/6J-Tyrc-2J/J, CBA/CaHN-Btkxid/J, CBA/CaJ, CBySmn.CB17-Prkdcscid/J, FVB/N-Tg(MMTVneu)202Mul/J, KK.Cg-Ay/J, MRL/MpJ, MRL/MpJ-Faslpr/J, SJL/J, SWR/J, B10.PL-H2u H2-T18a/(73NS)SnJ, NONcNZO5/LtJ, WR, BPH/2, BPL/1, FS, P/J, P/A, and PRO.

In certain embodiments, the mouse also includes variants with all genetically engineered (e.g. transgenic, knockout, knockin, knockdown, retroviral, viral) or chemically induced mutations (e.g., ENU, EMS also including archives of mutants); radiation induced mutations; spontaneous mutations / modifications maintained on mouse strains, particularly mutants derived from, for example, C57BL/6, 129, FVB, C3H, NOD, DBA/2, BALB/c, and CD-1, including congenic strains and recombinant congenic strains. Specific examples include: B6.129P2-Apoetm1Unc/J, B6.129S4-*Pdyn^{tmIUₜₑ}*/J, B6;129P2-*Pemt^{tmIJ}*/J, NOD.Cg *Prkdc^{scid}-B2m^{b}*/Dvs*, etc.*

In certain embodiments, the mouse is a mouse hybrid strain produced by crossing two inbred strains, including mixed inbred strains. Exemplary hybrid strain includes: NZBWF1/J, B6CBAF1/J, B6SJLF1/J, CB6F1/J, CByB6F1/J, PLSJLF1/J, WBB6F1/J-KitW/KitW-v, B6129PF1/J, CAF1/J, B6129PF2/J, B6129SF1/J, B6129SF2/J, B6AF1/J, B6C3F1/J, B6CBAF1/J, and B6SJLF1/J.

In any of the hybrid strains, the proportions may vary from 50:50 F1 to 99:1 F1.

In certain embodiments, the mouse is an outbred mouse, such as CD-1; ICR; Black Swiss; Swiss Webster; NIH Swiss; CF-1, and Nude mouse.

### 4. Genome Modification or Editing using CRISPR/Cas, TALEN, ZFN, or BuD

The present disclosure provides a powerful tool to modify or "edit" the genome of an animal of interest, such that the modified or edited genome of the animal can be passed along via germline transmission.

More specifically, genome modification or editing may be used to change the genomic sequence of an animal *(e.g.,* mammal) of interest, by, *e.g.,* introducing heterologous transgene or by inhibiting expression of a target endogenous gene. Such genetically modified animals can be used, for example, to establish relevant animal models that correspond to a specific genetic disease or disorder. Such animal models can be used to explore or study disease mechanism, progression, prevention, and treatment (e.g., drug screening and preclinical testing).

Illustrative (non-limiting) human diseases or disorders that can be represented in animal models include: various types of cancers, heart diseases, hypertension, metabolic and hormonal disorders, diabetes, obesity, osteoporosis, glaucoma, skin pigmentation diseases, blindness, deafness, neurodegenerative disorders (such as Huntington's or Alzheimer's disease), psychiatric disturbances (including anxiety and depression), and birth defects (such as cleft palate and anencephaly).

Most currently available animal models are made in mice, and they recreate some but may not be all aspects of any particular human disease. For certain disease that may be difficult to mimic using a mouse model, such as many neurodegenerative diseases (most of which involve cognitive deficits), the methods described herein may be used to generate animal models in non-human primates. For example, a transgenic model of Huntington's disease was recently developed using rhesus macaques that replicated some of the characteristic pathologies of the disorder as it occurs in humans (Yang *et al.,* 2008).

Genome editing may be performed using CRISPR technologies (see review by Gaj et al., Trends in Biotech., 31(7):397-405 (2013)). Such technologies enable one to manipulate virtually any gene in a diverse range of cell types and organisms, thus enabling a broad range of genetic modifications by inducing DNA double-strand (DSB) breaks that stimulate error-prone nonhomologous end joining (NHEJ) or homology-directed repair (HDR) at specific genomic locations.

CRISPR/Cas system can be used to efficiently induce targeted genetic alterations into the subject preimplantation embryos. CRISPR/Cas (CRISPR associated) systems or "Clustered Regulatory Interspaced Short Palindromic Repeats" are loci that contain multiple short direct repeats, and provide acquired immunity to bacteria and archaea. CRISPR systems rely on crRNA and tracrRNA for sequence-specific silencing of invading foreign DNA. The term "tracrRNA" stands for trans-activating chimeric RNA, which is noncoding RNA that promotes crRNA processing, and is required for activating RNA-guided cleavage by Cas9. CRISPR RNA or crRNA base pairs with tracrRNA to form a two-RNA structure that guides the Cas9 endonuclease to complementary DNA sites for cleavage.

Three types of CRISPR/Cas systems exist: in type II systems, Cas9 serves as an RNA-guided DNA endonuclease that cleaves DNA upon crRNA-tracrRNA target recognition. In bacteria, the CRISPR system provides acquired immunity against invading foreign DNA via RNA-guided DNA cleavage. The CRISPR/Cas system can be retargeted to cleave virtually any DNA sequence by redesigning the crRNA. Indeed, the CRISPR/Cas system has been shown to be directly portable to human cells by co-delivery of plasmids expressing the Cas9 endonuclease and the necessary crRNA components. These programmable RNA-guided DNA endonucleases have demonstrated multiplexed gene disruption capabilities and targeted integration in iPS cells, and can thus be used similarly in the subject methods.

Any CRISPR/Cas system, including the associated Cas proteins and RNA guide sequences, as well as their coding sequences (e.g., mRNA for Cas9 or coding sequence for the guide RNA) or vectors encompassing such coding sequences can be used with the methods described herein. See those described in US 2014-0068797 A1 and U.S. Pat. No. 8,697,359.

In the description that follows, the RNA guide sequence is also referred to as "DNA-targeting RNA," which comprises a targeting sequence and, together with a modifying polypeptide (such as Cas9), provides for site-specific modification of a target DNA and/or a polypeptide associated with the target DNA.

In the description that follows, the Cas9 type of protein in the CRISPR/Cas system is also referred to as "site-specific modifying polypeptides."

The DNA-targeting RNA directs the activities of an associated polypeptide *(e.g.,* a site-directed modifying polypeptide) to a specific target sequence within a target DNA. A subject DNA-targeting RNA comprises: a first segment (also referred to herein as a "DNA-targeting segment" or a "DNA-targeting sequence") and a second segment (also referred to herein as a "protein-binding segment" or a "protein-binding sequence").

The DNA-targeting segment of the DNA-targeting RNA comprises a nucleotide sequence that is complementary to a sequence in a target DNA. The DNA-targeting segment of a subject DNA-targeting RNA interacts with a target DNA in a sequence-specific manner via hybridization *(i.e.,* base pairing). As such, the nucleotide sequence of the DNA-targeting segment may vary and determines the location within the target DNA that the DNA-targeting RNA and the target DNA will interact. The DNA-targeting segment of the DNA-targeting RNA can be modified (e.g., by genetic engineering) to hybridize to any desired sequence within a target DNA.

The DNA-targeting segment can have a length of from about 12 nucleotides to about 100 nucleotides. For example, the DNA-targeting segment can have a length of from about 12 nucleotides (nt) to about 80 nt, from about 12 nt to about 50 nt, from about 12 nt to about 40 nt, from about 12 nt to about 30 nt, from about 12 nt to about 25 nt, from about 12 nt to about 20 nt, or from about 12 nt to about 19 nt. For example, the DNA-targeting segment can have a length of from about 19 nt to about 20 nt, from about 19 nt to about 25 nt, from about 19 nt to about 30 nt, from about 19 nt to about 35 nt, from about 19 nt to about 40 nt, from about 19 nt to about 45 nt, from about 19 nt to about 50 nt, from about 19 nt to about 60 nt, from about 19 nt to about 70 nt, from about 19 nt to about 80 nt, from about 19 nt to about 90 nt, from about 19 nt to about 100 nt, from about 20 nt to about 25 nt, from about 20 nt to about 30 nt, from about 20 nt to about 35 nt, from about 20 nt to about 40 nt, from about 20 nt to about 45 nt, from about 20 nt to about 50 nt, from about 20 nt to about 60 nt, from about 20 nt to about 70 nt, from about 20 nt to about 80 nt, from about 20 nt to about 90 nt, or from about 20 nt to about 100 nt. The nucleotide sequence (the DNA-targeting sequence) of the DNA-targeting segment that is complementary to a nucleotide sequence (target sequence) of the target DNA can have a length at least about 12 nt. For example, the DNA-targeting sequence of the DNA-targeting segment that is complementary to a target sequence of the target DNA can have a length at least about 12 nt, at least about 15 nt, at least about 18 nt, at least about 19 nt, at least about 20 nt, at least about 25 nt, at least about 30 nt, at least about 35 nt or at least about 40 nt. For example, the DNA-targeting sequence of the DNA-targeting segment that is complementary to a target sequence of the target DNA can have a length of from about 12 nucleotides (nt) to about 80 nt, from about 12 nt to about 50 nt, from about 12 nt to about 45 nt, from about 12 nt to about 40 nt, from about 12 nt to about 35 nt, from about 12 nt to about 30 nt, from about 12 nt to about 25 nt, from about 12 nt to about 20 nt, from about 12 nt to about 19 nt, from about 19 nt to about 20 nt, from about 19 nt to about 25 nt, from about 19 nt to about 30 nt, from about 19 nt to about 35 nt, from about 19 nt to about 40 nt, from about 19 nt to about 45 nt, from about 19 nt to about 50 nt, from about 19 nt to about 60 nt, from about 20 nt to about 25 nt, from about 20 nt to about 30 nt, from about 20 nt to about 35 nt, from about 20 nt to about 40 nt, from about 20 nt to about 45 nt, from about 20 nt to about 50 nt, or from about 20 nt to about 60 nt. The nucleotide sequence (the DNA-targeting sequence) of the DNA-targeting segment that is complementary to a nucleotide sequence (target sequence) of the target DNA can have a length at least about 12 nt.

In some embodiments, the DNA-targeting sequence of the DNA-targeting segment that is complementary to a target sequence of the target DNA is 20 nucleotides in length. In some embodiments, the DNA-targeting sequence of the DNA-targeting segment that is complementary to a target sequence of the target DNA is 19 nucleotides in length.

The percent complementarity between the DNA-targeting sequence of the DNA-targeting segment and the target sequence of the target DNA can be at least 60% *(e.g.,* at least 65%, at least 70%, at least 75%, at least 80%, at least 85%, at least 90%, at least 95%, at least 97%, at least 98%, at least 99%, or 100%). In some embodiments, the percent complementarity between the DNA-targeting sequence of the DNA-targeting segment and the target sequence of the target DNA is 100% over the seven contiguous 5'-most nucleotides of the target sequence of the complementary strand of the target DNA. In some embodiments, the percent complementarity between the DNA-targeting sequence of the DNA-targeting segment and the target sequence of the target DNA is at least 60% over about 20 contiguous nucleotides. In some embodiments, the percent complementarity between the DNA-targeting sequence of the DNA-targeting segment and the target sequence of the target DNA is 100% over the fourteen contiguous 5'-most nucleotides of the target sequence of the complementary strand of the target DNA and as low as 0% over the remainder. In such a embodiments, the DNA-targeting sequence can be considered to be 14 nucleotides in length. In some embodiments, the percent complementarity between the DNA-targeting sequence of the DNA-targeting segment and the target sequence of the target DNA is 100% over the seven contiguous 5'-most nucleotides of the target sequence of the complementary strand of the target DNA and as low as 0% over the remainder. In such a case, the DNA-targeting sequence can be considered to be 7 nucleotides in length.

The protein-binding segment of a subject DNA-targeting RNA interacts with a site-directed modifying polypeptide. The subject DNA-targeting RNA guides the bound polypeptide to a specific nucleotide sequence within target DNA via the above mentioned DNA-targeting segment. The protein-binding segment of a subject DNA-targeting RNA comprises two stretches of nucleotides that are complementary to one another. The complementary nucleotides of the protein-binding segment hybridize to form a double stranded RNA duplex (dsRNA).

A subject double-molecule DNA-targeting RNA comprises two separate RNA molecules. Each of the two RNA molecules of a subject double-molecule DNA-targeting RNA comprises a stretch of nucleotides that are complementary to one another such that the complementary nucleotides of the two RNA molecules hybridize to form the double stranded RNA duplex of the protein-binding segment.

In some embodiments, the duplex-forming segment of the activator-RNA is at least about 60% identical to one of the activator-RNA (tracrRNA) molecules (such as one set forth in SEQ ID NOs:431-562 of US 2014-0068797 A1), or a complement thereof, over a stretch of at least 8 contiguous nucleotides. For example, the duplex-forming segment of the activator-RNA (or the DNA encoding the duplex-forming segment of the activator-RNA) is at least about 60% identical, at least about 65% identical, at least about 70% identical, at least about 75% identical, at least about 80% identical, at least about 85% identical, at least about 90% identical, at least about 95% identical, at least about 98% identical, at least about 99% identical, or 100% identical, to one of the tracrRNA sequences set forth in SEQ ID NOs:431-562 of US 2014-0068797 A1, or a complement thereof, over a stretch of at least 8 contiguous nucleotides.

In some embodiments, the duplex-forming segment of the targeter-RNA is at least about 60% identical to one of the targeter-RNA (crRNA) sequences set forth in SEQ ID NOs:563-679 of US 2014-0068797 A1, or a complement thereof, over a stretch of at least 8 contiguous nucleotides. For example, the duplex-forming segment of the targeter-RNA (or the DNA encoding the duplex-forming segment of the targeter-RNA) is at least about 65% identical, at least about 70% identical, at least about 75% identical, at least about 80% identical, at least about 85% identical, at least about 90% identical, at least about 95% identical, at least about 98% identical, at least about 99% identical or 100% identical to one of the crRNA sequences set forth in SEQ ID NOs:563-679 of US 2014-0068797 A1, or a complement thereof, over a stretch of at least 8 contiguous nucleotides.

A two-molecule DNA-targeting RNA can be designed to allow for controlled *(i.e.,* conditional) binding of a targeter-RNA with an activator-RNA. Because a two-molecule DNA-targeting RNA is not functional unless both the activator-RNA and the targeter-RNA are bound in a functional complex with dCas9, a two-molecule DNA-targeting RNA can be inducible (e.g., drug inducible) by rendering the binding between the activator-RNA and the targeter-RNA to be inducible. As one non-limiting example, RNA aptamers can be used to regulate *(i.e.,* control) the binding of the activator-RNA with the targeter-RNA. Accordingly, the activator-RNA and/or the targeter-RNA can comprise an RNA aptamer sequence.

RNA aptamers are known in the art and are generally a synthetic version of a riboswitch. The terms "RNA aptamer" and "riboswitch" are used interchangeably herein to encompass both synthetic and natural nucleic acid sequences that provide for inducible regulation of the structure (and therefore the availability of specific sequences) of the RNA molecule of which they are part. RNA aptamers usually comprise a sequence that folds into a particular structure *(e.g.,* a hairpin), which specifically binds a particular drug *(e.g.,* a small molecule). Binding of the drug causes a structural change in the folding of the RNA, which changes a feature of the nucleic acid of which the aptamer is a part. As non-limiting examples: (i) an activator-RNA with an aptamer may not be able to bind to the cognate targeter-RNA unless the aptamer is bound by the appropriate drug; (ii) a targeter-RNA with an aptamer may not be able to bind to the cognate activator-RNA unless the aptamer is bound by the appropriate drug; and (iii) a targeter-RNA and an activator-RNA, each comprising a different aptamer that binds a different drug, may not be able to bind to each other unless both drugs are present. As illustrated by these examples, a two-molecule DNA-targeting RNA can be designed to be inducible.

Examples of aptamers and riboswitches can be found, for example, in: Nakamura et al., Genes Cells, 2012 May, 17(5):344-364; Vavalle et al., Future Cardiol., 2012 May, 8(3):371-382; Citartan et al., Biosens. Bioelectron., 2012 Apr. 15, 34(1): 1-11; and Liberman et al., Wiley Interdiscip. Rev. RNA, 2012 May-June, 3(3):369-384.

Non-limiting examples of nucleotide sequences that can be included in a two-molecule DNA-targeting RNA include either of the sequences set forth in SEQ ID NOs:431-562 of US 2014-0068797 A1, or complements thereof pairing with any sequences set forth in SEQ ID NOs:563-679 of US 2014-0068797 A1, or complements thereof that can hybridize to form a protein binding segment.

A single-molecule DNA-targeting RNA comprises two stretches of nucleotides (a targeter-RNA and an activator-RNA) that are complementary to one another, are covalently linked by intervening nucleotides ("linkers" or "linker nucleotides"), and hybridize to form the double stranded RNA duplex (dsRNA duplex) of the protein-binding segment, thus resulting in a stem-loop structure. The targeter-RNA and the activator-RNA can be covalently linked via the 3' end of the targeter-RNA and the 5' end of the activator-RNA. Alternatively, targeter-RNA and the activator-RNA can be covalently linked via the 5' end of the targeter-RNA and the 3' end of the activator-RNA.

The linker of a single-molecule DNA-targeting RNA can have a length of from about 3 nucleotides to about 100 nucleotides. For example, the linker can have a length of from about 3 nucleotides (nt) to about 90 nt, from about 3 nucleotides (nt) to about 80 nt, from about 3 nucleotides (nt) to about 70 nt, from about 3 nucleotides (nt) to about 60 nt, from about 3 nucleotides (nt) to about 50 nt, from about 3 nucleotides (nt) to about 40 nt, from about 3 nucleotides (nt) to about 30 nt, from about 3 nucleotides (nt) to about 20 nt or from about 3 nucleotides (nt) to about 10 nt. For example, the linker can have a length of from about 3 nt to about 5 nt, from about 5 nt to about 10 nt, from about 10 nt to about 15 nt, from about 15 nt to about 20 nt, from about 20 nt to about 25 nt, from about 25 nt to about 30 nt, from about 30 nt to about 35 nt, from about 35 nt to about 40 nt, from about 40 nt to about 50 nt, from about 50 nt to about 60 nt, from about 60 nt to about 70 nt, from about 70 nt to about 80 nt, from about 80 nt to about 90 nt, or from about 90 nt to about 100 nt. In some embodiments, the linker of a single-molecule DNA-targeting RNA is 4 nt.

An exemplary single-molecule DNA-targeting RNA comprises two complementary stretches of nucleotides that hybridize to form a dsRNA duplex. In some embodiments, one of the two complementary stretches of nucleotides of the single-molecule DNA-targeting RNA (or the DNA encoding the stretch) is at least about 60% identical to one of the activator-RNA (tracrRNA) molecules set forth in SEQ ID NOs:431-562 of US 2014-0068797 A1, or a complement thereof, over a stretch of at least 8 contiguous nucleotides. For example, one of the two complementary stretches of nucleotides of the single-molecule DNA-targeting RNA (or the DNA encoding the stretch) is at least about 65% identical, at least about 70% identical, at least about 75% identical, at least about 80% identical, at least about 85% identical, at least about 90% identical, at least about 95% identical, at least about 98% identical, at least about 99% identical or 100% identical to one of the tracrRNA sequences set forth in SEQ ID NOs:431-562 of US 2014-0068797 A1, or a complement thereof, over a stretch of at least 8 contiguous nucleotides.

In some embodiments, one of the two complementary stretches of nucleotides of the single-molecule DNA-targeting RNA (or the DNA encoding the stretch) is at least about 60% identical to one of the targeter-RNA (crRNA) sequences set forth in SEQ ID NOs:563-679, or a complement thereof, over a stretch of at least 8 contiguous nucleotides. For example, one of the two complementary stretches of nucleotides of the single-molecule DNA-targeting RNA (or the DNA encoding the stretch) is at least about 65% identical, at least about 70% identical, at least about 75% identical, at least about 80% identical, at least about 85% identical, at least about 90% identical, at least about 95% identical, at least about 98% identical, at least about 99% identical or 100% identical to one of the crRNA sequences set forth in SEQ ID NOs:563-679 of US 2014-0068797 A1, or a complement thereof, over a stretch of at least 8 contiguous nucleotides.

Appropriate naturally occurring cognate pairs of crRNAs and tracrRNAs can be routinely determined for SEQ ID NOs:431-679 of US 2014-0068797 A1 by taking into account the species name and base-pairing (for the dsRNA duplex of the protein-binding domain) when determining appropriate cognate pairs.

With regard to both a single-molecule DNA-targeting RNA and to a double-molecule DNA-targeting RNA, artificial sequences that share very little (roughly 50% identity) with naturally occurring a tracrRNAs and crRNAs can function with Cas9 to cleave target DNA as long as the structure of the protein-binding domain of the DNA-targeting RNA is conserved. Thus, RNA folding structure of a naturally occurring protein-binding domain of a DNA-targeting RNA can be taken into account in order to design artificial protein-binding domains (either two-molecule or single-molecule versions). As a non-limiting example, functional artificial DNA-targeting RNA can be designed based on the structure of the protein-binding segment of the naturally occurring DNA-targeting (e.g., including the same number of base pairs along the RNA duplex and including the same "bulge" region as present in the naturally occurring RNA). As structures can readily be produced by one of ordinary skill in the art for any naturally occurring crRNA:tracrRNA pair from any species (see SEQ ID NOs:431-679 of US 2014-0068797 A1 for crRNA and tracrRNA sequences from a wide variety of species), an artificial DNA-targeting-RNA can be designed to mimic the natural structure for a given species when using the Cas9 (or a related Cas9) from that species. Thus, a suitable DNA-targeting RNA can be an artificially designed RNA (non-naturally occurring) comprising a protein-binding domain that was designed to mimic the structure of a protein-binding domain of a naturally occurring DNA-targeting RNA. (see SEQ ID NOs:431-679 of US 2014-0068797 A1, taking into account the species name when determining appropriate cognate pairs).

The protein-binding segment can have a length of from about 10 nucleotides to about 100 nucleotides. For example, the protein-binding segment can have a length of from about 15 nucleotides (nt) to about 80 nt, from about 15 nt to about 50 nt, from about 15 nt to about 40 nt, from about 15 nt to about 30 nt or from about 15 nt to about 25 nt.

Also with regard to both a single-molecule DNA-targeting RNA and to a double-molecule DNA-targeting RNA, the dsRNA duplex of the protein-binding segment can have a length from about 6 base pairs (bp) to about 50 bp. For example, the dsRNA duplex of the protein-binding segment can have a length from about 6 bp to about 40 bp, from about 6 bp to about 30 bp, from about 6 bp to about 25 bp, from about 6 bp to about 20 bp, from about 6 bp to about 15 bp, from about 8 bp to about 40 bp, from about 8 bp to about 30 bp, from about 8 bp to about 25 bp, from about 8 bp to about 20 bp or from about 8 bp to about 15 bp. For example, the dsRNA duplex of the protein-binding segment can have a length from about from about 8 bp to about 10 bp, from about 10 bp to about 15 bp, from about 15 bp to about 18 bp, from about 18 bp to about 20 bp, from about 20 bp to about 25 bp, from about 25 bp to about 30 bp, from about 30 bp to about 35 bp, from about 35 bp to about 40 bp, or from about 40 bp to about 50 bp. In some embodiments, the dsRNA duplex of the protein-binding segment has a length of 36 base pairs. The percent complementarity between the nucleotide sequences that hybridize to form the dsRNA duplex of the protein-binding segment can be at least about 60%. For example, the percent complementarity between the nucleotide sequences that hybridize to form the dsRNA duplex of the protein-binding segment can be at least about 65%, at least about 70%, at least about 75%, at least about 80%, at least about 85%, at least about 90%, at least about 95%, at least about 98%, or at least about 99%. In some cases, the percent complementarity between the nucleotide sequences that hybridize to form the dsRNA duplex of the protein-binding segment is 100%.

A DNA-targeting RNA and a site-directed modifying polypeptide form a complex. The DNA-targeting RNA provides target specificity to the complex by comprising a nucleotide sequence that is complementary to a sequence of a target DNA (as noted above). The site-directed modifying polypeptide of the complex provides the site-specific activity. In other words, the site-directed modifying polypeptide is guided to a DNA sequence *(e.g.* a chromosomal sequence or an extrachromosomal sequence, e.g. an episomal sequence, a minicircle sequence, a mitochondrial sequence, a chloroplast sequence, etc.) by virtue of its association with at least the protein-binding segment of the DNA-targeting RNA.

A site-directed modifying polypeptide modifies target DNA (e.g., cleavage or methylation of target DNA) and/or a polypeptide associated with target DNA *(e.g.,* methylation or acetylation of a histone tail). A site-directed modifying polypeptide is also referred to herein as a "site-directed polypeptide" or an "RNA binding site-directed modifying polypeptide."

In some embodiments, the site-directed modifying polypeptide is a naturally-occurring modifying polypeptide. In other cases, the site-directed modifying polypeptide is not a naturally-occurring polypeptide *(e.g.,* a chimeric polypeptide as discussed below or a naturally-occurring polypeptide that is modified, e.g., mutation, deletion, insertion).

Exemplary naturally-occurring site-directed modifying polypeptides are set forth in SEQ ID NOs: 1-255 of US 2014-0068797 A1 as a non-limiting and non-exhaustive list of naturally occurring Cas9/Csn1 endonucleases. These naturally occurring polypeptides, as disclosed herein, bind a DNA-targeting RNA, are thereby directed to a specific sequence within a target DNA, and cleave the target DNA to generate a double strand break.

A site-directed modifying polypeptide comprises two portions, an RNA-binding portion and an activity portion. In some embodiments, a site-directed modifying polypeptide comprises: (i) an RNA-binding portion that interacts with a DNA-targeting RNA, wherein the DNA-targeting RNA comprises a nucleotide sequence that is complementary to a sequence in a target DNA; and (ii) an activity portion that exhibits site-directed enzymatic activity (e.g., activity for DNA methylation, activity for DNA cleavage, activity for histone acetylation, activity for histone methylation, *etc.),* wherein the site of enzymatic activity is determined by the DNA-targeting RNA.

In other embodiments, a site-directed modifying polypeptide comprises: (i) an RNA-binding portion that interacts with a DNA-targeting RNA, wherein the DNA-targeting RNA comprises a nucleotide sequence that is complementary to a sequence in a target DNA; and (ii) an activity portion that modulates transcription within the target DNA *(e.g.,* to increase or decrease transcription), wherein the site of modulated transcription within the target DNA is determined by the DNA-targeting RNA.

In some embodiments, a site-directed modifying polypeptide has enzymatic activity that modifies target DNA (e.g., nuclease activity, methyltransferase activity, demethylase activity, DNA repair activity, DNA damage activity, deamination activity, dismutase activity, alkylation activity, depurination activity, oxidation activity, pyrimidine dimer forming activity, integrase activity, transposase activity, recombinase activity, polymerase activity, ligase activity, helicase activity, photolyase activity or glycosylase activity).

In other cases, a subject site-directed modifying polypeptide has enzymatic activity that modifies a polypeptide *(e.g.,* a histone) associated with target DNA *(e.g.,* methyltransferase activity, demethylase activity, acetyltransferase activity, deacetylase activity, kinase activity, phosphatase activity, ubiquitin ligase activity, deubiquitinating activity, adenylation activity, deadenylation activity, SUMOylating activity, deSUMOylating activity, ribosylation activity, deribosylation activity, myristoylation activity or demyristoylation activity).

Exemplary site-directed modifying polypeptides are further provided below.

Specifically, in some embodiments, the site-directed modifying polypeptide comprises an amino acid sequence having at least about 75%, at least about 80%, at least about 85%, at least about 90%, at least about 95%, at least about 99%, or 100%, amino acid sequence identity to amino acids 7-166 or 731-1003 of the Cas9/Csn1 amino acid sequence depicted in FIG. 3 of US 2014-0068797 A1, or to the corresponding portions in any of the amino acid sequences set forth as SEQ ID NOs: 1-256 and 795-1346 of US 2014-0068797 A1.

In some embodiments, a nucleic acid *(e.g.,* a DNA-targeting RNA) comprises one or more modifications, *e.g.,* a base modification, a backbone modification, *etc.,* to provide the nucleic acid with a new or enhanced feature (e.g., improved stability). As is known in the art, a nucleoside is a base-sugar combination. The base portion of the nucleoside is normally a heterocyclic base. The two most common classes of such heterocyclic bases are the purines and the pyrimidines. Nucleotides are nucleosides that further include a phosphate group covalently linked to the sugar portion of the nucleoside. For those nucleosides that include a pentofuranosyl sugar, the phosphate group can be linked to the 2', the 3', or the 5' hydroxyl moiety of the sugar. In forming oligonucleotides, the phosphate groups covalently link adjacent nucleosides to one another to form a linear polymeric compound. In turn, the respective ends of this linear polymeric compound can be further joined to form a circular compound, however, linear compounds are generally suitable. In addition, linear compounds may have internal nucleotide base complementarity and may therefore fold in a manner as to produce a fully or partially double-stranded compound. Within oligonucleotides, the phosphate groups are commonly referred to as forming the internucleoside backbone of the oligonucleotide. The normal linkage or backbone of RNA and DNA is a 3' to 5' phosphodiester linkage.

Examples of suitable nucleic acids containing modifications include nucleic acids containing modified backbones or non-natural internucleoside linkages. Nucleic acids (having modified backbones include those that retain a phosphorus atom in the backbone and those that do not have a phosphorus atom in the backbone.

Suitable modified oligonucleotide backbones containing a phosphorus atom therein include, for example, phosphorothioates, chiral phosphorothioates, phosphorodithioates, phosphotriesters, aminoalkylphosphotriesters, methyl and other alkyl phosphonates including 3'-alkylene phosphonates, 5'-alkylene phosphonates and chiral phosphonates, phosphinates, phosphoramidates including 3'-amino phosphoramidate and aminoalkylphosphoramidates, phosphorodiamidates, thionophosphoramidates, thionoalkylphosphonates, thionoalkylphosphotriesters, selenophosphates and boranophosphates having normal 3'-5' linkages, 2'-5' linked analogs of these, and those having inverted polarity wherein one or more internucleotide linkages is a 3' to 3', 5' to 5' or 2' to 2' linkage. Suitable oligonucleotides having inverted polarity comprise a single 3' to 3' linkage at the 3'-most internucleotide linkage, *i.e.,* a single inverted nucleoside residue which may be a basic (the nucleobase is missing or has a hydroxyl group in place thereof). Various salts (such as, for example, potassium or sodium), mixed salts and free acid forms are also included.

In some embodiments, a subject nucleic acid comprises one or more phosphorothioate and/or heteroatom internucleoside linkages, in particular -CH₂-NH-O-CH₂-, -CH₂-N(CH₃)-O-CH₂- (known as a methylene (methylimino) or MMI backbone), -CH₂-O-N(CH₃)-CH₂-, - CH₂-N(CH₃)-N(CH₃)-CH₂- and -O-N(CH₃)-CH₂-CH₂- (wherein the native phosphodiester internucleotide linkage is represented as -O-P(=O)(OH)-O-CH₂-). MMI type internucleoside linkages are disclosed in the above referenced U.S. Pat. No. 5,489,677. Suitable amide internucleoside linkages are disclosed in t U.S. Pat. No. 5,602,240.

Also suitable are nucleic acids having morpholino backbone structures as described in, *e.g.,* U.S. Pat. No. 5,034,506. For example, in some embodiments, a subject nucleic acid comprises a 6-membered morpholino ring in place of a ribose ring. In some of these embodiments, a phosphorodiamidate or other non-phosphodiester internucleoside linkage replaces a phosphodiester linkage.

Suitable modified polynucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; riboacetyl backbones; alkene containing backbones; sulfamate backbones; methyleneimino and methylenehydrazino backbones; sulfonate and sulfonamide backbones; amide backbones; and others having mixed N, O, S and CH₂ component parts.

A subject nucleic acid can also be a nucleic acid mimetic. The term "mimetic" as it is applied to polynucleotides is intended to include polynucleotides wherein only the furanose ring or both the furanose ring and the internucleotide linkage are replaced with non-furanose groups, replacement of only the furanose ring is also referred to in the art as being a sugar surrogate. The heterocyclic base moiety or a modified heterocyclic base moiety is maintained for hybridization with an appropriate target nucleic acid. One such nucleic acid, a polynucleotide mimetic that has been shown to have excellent hybridization properties, is referred to as a peptide nucleic acid (PNA). The backbone in PNA compounds is two or more linked aminoethylglycine units which gives PNA an amide containing backbone. The heterocyclic base moieties are bound directly or indirectly to aza nitrogen atoms of the amide portion of the backbone. Representative U.S. patents that describe the preparation of PNA compounds include, but are not limited to: U.S. Pat. Nos. 5,539,082; 5,714,331; and 5,719,262.

Another class of polynucleotide mimetic that has been studied is based on linked morpholino units (morpholino nucleic acid) having heterocyclic bases attached to the morpholino ring. A number of linking groups have been reported that link the morpholino monomeric units in a morpholino nucleic acid. One class of linking groups has been selected to give a non-ionic oligomeric compound. The non-ionic morpholino-based oligomeric compounds are less likely to have undesired interactions with cellular proteins. Morpholino-based polynucleotides are non-ionic mimics of oligonucleotides which are less likely to form undesired interactions with cellular proteins (Dwaine A. Braasch and David R. Corey, Biochemistry, 41(14):4503-4510 (2002)). Morpholino-based polynucleotides are disclosed in U.S. Pat. No. 5,034,506. A variety of compounds within the morpholino class of polynucleotides have been prepared, having a variety of different linking groups joining the monomeric subunits.

A further class of polynucleotide mimetic is referred to as cyclohexenyl nucleic acids (CeNA). The furanose ring normally present in a DNA/RNA molecule is replaced with a cyclohexenyl ring. CeNA DMT protected phosphoramidite monomers have been prepared and used for oligomeric compound synthesis following classical phosphoramidite chemistry. Fully modified CeNA oligomeric compounds and oligonucleotides having specific positions modified with CeNA have been prepared and studied (see Wang et al., J. Am. Chem. Soc., 122:8595-8602 (2000)). In general the incorporation of CeNA monomers into a DNA chain increases its stability of a DNA/RNA hybrid. CeNA oligoadenylates formed complexes with RNA and DNA complements with similar stability to the native complexes. The study of incorporating CeNA structures into natural nucleic acid structures was shown by NMR and circular dichroism to proceed with easy conformational adaptation.

A further modification includes Locked Nucleic Acids (LNAs) in which the 2'-hydroxyl group is linked to the 4' carbon atom of the sugar ring thereby forming a 2'-C,4'-C-oxymethylene linkage thereby forming a bicyclic sugar moiety. The linkage can be a methylene (-CH₂-), group bridging the 2' oxygen atom and the 4' carbon atom wherein n is 1 or 2 (Singh et al., Chem. Commun., 4:455-456 (1998)). LNA and LNA analogs display very high duplex thermal stabilities with complementary DNA and RNA (Tm=+3 to +10°C), stability towards 3'-exonucleolytic degradation and good solubility properties. Potent and nontoxic antisense oligonucleotides containing LNAs have been described (Wahlestedt et al., Proc. Natl. Acad. Sci. U.S.A., 97:5633-5638 (2000)).

The synthesis and preparation of the LNA monomers adenine, cytosine, guanine, 5-methyl-cytosine, thymine and uracil, along with their oligomerization, and nucleic acid recognition properties have been described (Koshkin et al., Tetrahedron, 54:3607-3630 (1998)). LNAs and preparation thereof are also described in WO 98/39352 and WO 99/14226.

A nucleic acid can also include one or more substituted sugar moieties. Suitable polynucleotides comprise a sugar substituent group selected from: OH; F; O-, S-, or N-alkyl; O-, S-, or N-alkenyl; O-, S- or N-alkynyl; or O-alkyl-O-alkyl, wherein the alkyl, alkenyl and alkynyl may be substituted or unsubstituted C₁₋₁₀ alkyl or C₂₋₁₀ alkenyl and alkynyl. Particularly suitable are O((CH₂)ₙO)ₘCH₃, O(CH₂)ₙOCH₃, O(CH₂)ₙNH₂, O(CH₂)ₙCH₃, O(CH₂)ₙONH₂, and O(CH₂)ₙON((CH₂)ₙCH₃)₂, where n and m are from 1 to about 10. Other suitable polynucleotides comprise a sugar substituent group selected from: C₁₋₁₀ lower alkyl, substituted lower alkyl, alkenyl, alkynyl, alkaryl, aralkyl, O-alkaryl or O-aralkyl, SH, SCH₃, OCN, Cl, Br, CN, CF₃, OCF₃, SOCH₃, SO₂CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. A suitable modification includes 2'-methoxyethoxy (2'-O--CH2CH2OCH3, also known as 2'-O-(2-methoxyethyl) or 2'-MOE) (Martin et al., Helv. Chim. Acta, 78:486-504 (1995)) *i.e.,* an alkoxyalkoxy group. A further suitable modification includes 2'-dimethylaminooxyethoxy, *i.e.,* a 0(CH₂)₂ON(CH₃)₂ group, also known as 2'-DMAOE, as described in examples hereinbelow, and 2'-dimethylaminoethoxyethoxy (also known in the art as 2'-O-dimethyl-amino-ethoxyethyl or 2'-DMAEOE), *i.e.,* 2'-O-CH₂-O-CH₂-N(CH₃)₂.

Other suitable sugar substituent groups include methoxy (-O-CH₃), aminopropoxy (-OCH₂CH₂CH₂NH₂), allyl (-CH₂-CH=CH₂), -O-allyl (-O-CH₂CH=CH₂) and fluoro (F). 2'sugar substituent groups may be in the arabino (up) position or ribo (down) position. A suitable 2'-arabino modification is 2'-F. Similar modifications may also be made at other positions on the oligomeric compound, particularly the 3' position of the sugar on the 3' terminal nucleoside or in 2'-5' linked oligonucleotides and the 5' position of 5' terminal nucleotide. Oligomeric compounds may also have sugar mimetics such as cyclobutyl moieties in place of the pentofuranosyl sugar.

A subject nucleic acid may also include nucleobase (often referred to in the art simply as "base") modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C=C-CH₃) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-aminoadenine, 8-azaguanine and 8-azaadenine, 7-deazaguanine and 7-deazaadenine and 3-deazaguanine and 3-deazaadenine. Further modified nucleobases include tricyclic pyrimidines such as phenoxazine cytidine(1H-pyrimido(5,4-b)(1,4)benzoxazin-2(3H)-one), phenothiazine cytidine (1H-pyrimido(5,4-b)(1,4)benzothiazin-2(3H)-one), G-clamps such as a substituted phenoxazine cytidine (e.g., 9-(2-aminoethoxy)-H-pyrimido(5,4-(b) (1,4)benzoxazin-2(3H)-one), carbazole cytidine (2H-pyrimido(4,5-b)indol-2-one), pyridoindole cytidine (H-pyrido(3',':4,5)pyrrolo[2,3-d]pyrimidin-2-one).

Heterocyclic base moieties may also include those in which the purine or pyrimidine base is replaced with other heterocycles, for example 7-deaza-adenine, 7-deazaguanosine, 2-aminopyridine and 2-pyridone. Further nucleobases include those disclosed in U.S. Pat. No. 3,687,808, those disclosed in The Concise Encyclopedia of Polymer Science and Engineering, pages 858-859, Kroschwitz, J. I., ed. John Wiley & Sons, 1990; those disclosed by Englisch et al., Angewandte Chemie, International Edition, 1991, 30:613; and those disclosed by Sanghvi, Y. S., Chapter 15, Antisense Research and Applications, pages 289-302, Crooke, S. T. and Lebleu, B., ed., CRC Press, 1993. Certain of these nucleobases are useful for increasing the binding affinity of an oligomeric compound. These include 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2°C. (Sanghvi et al., eds., Antisense Research and Applications, CRC Press, Boca Raton, 1993, pp. 276-278) and are suitable base substitutions, e.g., when combined with 2'-O-methoxyethyl sugar modifications.

Another possible modification of a subject nucleic acid involves chemically linking to the polynucleotide one or more moieties or conjugates which enhance the activity, cellular distribution or cellular uptake of the oligonucleotide. These moieties or conjugates can include conjugate groups covalently bound to functional groups such as primary or secondary hydroxyl groups. Conjugate groups include, but are not limited to, intercalators, reporter molecules, polyamines, polyamides, polyethylene glycols, polyethers, groups that enhance the pharmacodynamic properties of oligomers, and groups that enhance the pharmacokinetic properties of oligomers. Suitable conjugate groups include, but are not limited to, cholesterols, lipids, phospholipids, biotin, phenazine, folate, phenanthridine, anthraquinone, acridine, fluoresceins, rhodamines, coumarins, and dyes. Groups that enhance the pharmacodynamic properties include groups that improve uptake, enhance resistance to degradation, and/or strengthen sequence-specific hybridization with the target nucleic acid. Groups that enhance the pharmacokinetic properties include groups that improve uptake, distribution, metabolism or excretion of a subject nucleic acid.

Conjugate moieties include but are not limited to lipid moieties such as a cholesterol moiety (Letsinger et al., Proc. Natl. Acad. Sci. USA, 86:6553-6556 (1989)); cholic acid (Manoharan et al., Bioorg. Med. Chem. Let., 4:1053-1060 (1994)); a thioether, *e.g.,* hexyl-5-tritylthiol (Manoharan et al., Ann. N.Y. Acad. Sci., 660:306-309 (1992)); Manoharan et al., Bioorg. Med. Chem. Let., 3:2765-2770 (1993)); a thiocholesterol (Oberhauser et al., Nucl. Acids Res., 20:533-538 (1992)); an aliphatic chain, *e.g.,* dodecandiol or undecyl residues (Saison-Behmoaras et al., EMBO J., 10:1111-1118 (1991)); Kabanov et al., FEBS Lett., 259:327-330 (1990)); Svinarchuk et al., Biochimie, 75:49-54 (1993)); a phospholipid, *e.g.,* dihexadecyl-rac-glycerol or triethylammonium 1,2-di-O-hexadecyl-rac-glycero-3-H-phosphonate (Manoharan et al., Tetrahedron Lett., 36:3651-3654 (1995)); Shea et al., Nucl. Acids Res., 18:3777-3783 (1990)); a polyamine or a polyethylene glycol chain (Manoharan et al., Nucleosides & Nucleotides, 14:969-973 (1995)); or adamantane acetic acid (Manoharan et al., Tetrahedron Lett., 36:3651-3654 (1995)); a palmityl moiety (Mishra et al., Biochim. Biophys. Acta, 1264:229-237 (1995)); or an octadecylamine or hexylamino-carbonyloxycholesterol moiety (Crooke et al., J. Pharmacol. Exp. Ther., 277:923-937 (1996)).

A conjugate may include a "Protein Transduction Domain" or PTD (also known as a CPP-cell penetrating peptide), which may refer to a polypeptide, polynucleotide, carbohydrate, or organic or inorganic compound that facilitates traversing a lipid bilayer, micelle, cell membrane, organelle membrane, or vesicle membrane. A PTD attached to another molecule, which can range from a small polar molecule to a large macromolecule and/or a nanoparticle, facilitates the molecule traversing a membrane, for example going from extracellular space to intracellular space, or cytosol to within an organelle. In some embodiments, a PTD is covalently linked to the amino terminus of an exogenous polypeptide *(e.g.,* a site-directed modifying polypeptide). In some embodiments, a PTD is covalently linked to the carboxyl terminus of an exogenous polypeptide *(e.g.,* a site-directed modifying polypeptide). In some embodiments, a PTD is covalently linked to a nucleic acid *(e.g.,* a DNA-targeting RNA, a polynucleotide encoding a DNA-targeting RNA, a polynucleotide encoding a site-directed modifying polypeptide, *etc.).* Exemplary PTDs include but are not limited to a minimal undecapeptide protein transduction domain (corresponding to residues 47-57 of HIV-1 TAT comprising YGRKKRRQRRR); a polyarginine sequence comprising a number of arginines sufficient to direct entry into a cell *(e.g.,* 3, 4, 5, 6, 7, 8, 9, 10, or 10-50 arginines); a VP22 domain (Zender et al., Cancer Gene Ther., 9(6):489-496 (2002)); an Drosophila Antennapedia protein transduction domain (Noguchi et al., Diabetes, 52(7): 1732-1737 (2003)); a truncated human calcitonin peptide (Trehin et al., Pharm. Research, 21:1248-1256 (2004)); polylysine (Wender et al., Proc. Natl. Acad. Sci. USA, 97:13003-13008 (2000)); RRQRRTSKLMKR; Transportan GWTLNSAGYLLGKINLKALAALAKKIL; KALAWEAKLAKALAKALAKHLAKALAKALKCEA; and RQIKIWFQNRRMKWKK. Exemplary PTDs include but are not limited to, YGRKKRRQRRR, RKKRRQRRR; an arginine homopolymer of from 3 arginine residues to 50 arginine residues; Exemplary PTD domain amino acid sequences include, but are not limited to, any of the following: YGRKKRRQRRR; RKKRRQRR; YARAAARQARA; THRLPRRRRRR; and GGRRARRRRRR. In some embodiments, the PTD is an activatable CPP (ACPP) (Aguilera et al., Integr. Biol. (Camb), 1(5-6):371-381 (June, 2009)). ACPPs comprise a polycationic CPP *(e.g.,* Arg9 or "R9") connected via a cleavable linker to a matching polyanion *(e.g.,* Glu9 or "E9"), which reduces the net charge to nearly zero and thereby inhibits adhesion and uptake into cells. Upon cleavage of the linker, the polyanion is released, locally unmasking the polyarginine and its inherent adhesiveness, thus "activating" the ACPP to traverse the membrane.

Exemplary DNA-Targeting RNAs are further described below.

In some embodiments, a suitable DNA-targeting RNA comprises two separate RNA polynucleotide molecules. The first of the two separate RNA polynucleotide molecules (the activator-RNA) comprises a nucleotide sequence having at least about 60%, at least about 65%, at least about 70%, at least about 75%, at least about 80%, at least about 85%, at least about 90%, at least about 95%, at least about 98%, at least about 99%, or 100% nucleotide sequence identity over a stretch of at least 8 contiguous nucleotides to any one of the nucleotide sequences set forth in SEQ ID NOs:431-562, or complements thereof. The second of the two separate RNA polynucleotide molecules (the targeter-RNA) comprises a nucleotide sequence having at least about 60%, at least about 65%, at least about 70%, at least about 75%, at least about 80%, at least about 85%, at least about 90%, at least about 95%, at least about 98%, at least about 99%, or 100% nucleotide sequence identity over a stretch of at least 8 contiguous nucleotides to any one of the nucleotide sequences set forth in SEQ ID NOs:563-679 of US 2014-0068797, or complements thereof.

In some embodiments, a suitable DNA-targeting RNA is a single RNA polynucleotide and comprises a first nucleotide sequence having at least about 60%, at least about 65%, at least about 70%, at least about 75%, at least about 80%, at least about 85%, at least about 90%, at least about 95%, at least about 98%, at least about 99%, or 100% nucleotide sequence identity over a stretch of at least 8 contiguous nucleotides to any one of the nucleotide sequences set forth in SEQ ID NOs:431-562 of US 2014-0068797, and a second nucleotide sequence having at least about 60%, at least about 65%, at least about 70%, at least about 75%, at least about 80%, at least about 85%, at least about 90%, at least about 95%, at least about 98%, at least about 99%, or 100% nucleotide sequence identity over a stretch of at least 8 contiguous nucleotides to any one of the nucleotide sequences set forth in SEQ ID NOs: 463-679 of US 2014-0068797.

In some embodiments, the DNA-targeting RNA is a double-molecule DNA-targeting RNA and the targeter-RNA comprises the sequence 5'GUUUUAGAGCUA-3' linked at its 5' end to a stretch of nucleotides that are complementary to a target DNA. In some embodiments, the DNA-targeting RNA is a double-molecule DNA-targeting RNA and the activator-RNA comprises the sequence 5' UAGCAAGUUAAAAUAAGGCUAGUCCG-3'.

In some embodiments, the DNA-targeting RNA is a single-molecule DNA-targeting RNA and comprises the sequence 5'-GUUUUAGAGCUA-linker-UAGCAAGUUAAAAUAAGGCUAGUCCG-3' linked at its 5' end to a stretch of nucleotides that are complementary to a target DNA (where "linker" denotes any a linker nucleotide sequence that can comprise any nucleotide sequence). Other exemplary single-molecule DNA-targeting RNAs include those set forth in SEQ ID NOs: 680-682 of US 2014-0068797.

In certain embodiments, the site-specific modifying polypeptides (e.g., Cas9) may be constitutively or conditionally (e.g., tissue- or cell type-specifically or inducibly) expressed as a transgene in an animal of interest, such as a mouse or a rat, such that only the DNA-targeting RNA or guide sequence needs to be introduced into gametes or preimplantation embryos obtained from such animals to provide a functional CRISPR/Cas system in the gametes or preimplantation embryos. For example, see the Cre-dependent Cas9 knockin mouse described in Platt et al., RISPR-Cas9 Knockin Mice for Genome Editing and Cancer Modeling, Cell http://dx dot doi dot org slash 10.1016 slash j dot cell dot 2014.09.014, in which successful genome editing was demonstrated in neurons, immune cells, and endothelial cells, using guide RNA delivered by adeno-associated virus (AAV)-, lentivirus-, or particle-mediated delivery methods. The Cas9 type of transgene can be driven by, for example, a ubiquitous promoter (such as the CAG promoter), and may be rendered inducible by using the Cre-loxP system (e.g., with cell type- or tissue-specific Cre drivers) or equivalents or variants thereof well known in the art.

In certain embodiments, the Cas9-expressing animal is fertile, has normal litter sizes, presents no morphological abnormalities, and/or is able to breed to homozygosity.

Such site-specific modifying polypeptide-expressing animals (e.g., mouse or rat), coupled with the high efficiency, high throughout electroporation methods described herein, may be used in *in vivo* high-throughput genetic screen to, for example, identify genes involved in any of many important biological processes such as tumor suppression, stem-cell renewal, host determinants of virus replication, and regulators of oncogenic growth, and may be directly combined with genome-wide or targeted sgRNA library to uncover novel biology.

### EXAMPLES

The examples described herein are for illustrative purpose only, and are not limiting. The conditions described in the examples, however, may have general applicability and constitute specific embodiments of the described invention that are contemplated to be combinable with any one or more other embodiments of the invention described herein.

Genetically modified mice serve as important models for studying gene function and human diseases, and predominantly have been created using conventional gene targeting or transgenic technologies. In a typical conventional gene targeting experiment, the desired genetic modification is first introduced into the mouse embryonic stem (ES) cells through homologous recombination, and the clonally derived ES cell clones carrying the intended mutation are isolated (Capecchi, 2005). Targeted ES cells are subsequently injected into wild-type blastocysts. Some of the targeted ES cells may contribute to the germline of the resulting chimeric animals, thus generating mice containing the targeted gene modification (Capecchi, 2005).

Although effective, gene targeting is costly and time-consuming, and heavily relies on the availability and performance of a germline competent ES cell line.

To circumvent the need of a germline competent ES cell line, alternative methodologies have been developed using site-specific DNA endonucleases, including zinc-finger nucleases (ZFNs) (Urnov *et al.,* 2010) and transcription activator-like effector nucleases (TALENs) (Bogdanove and Voytas, 2011), which could be injected directly into the one-cell embryos to generate animals with specific gene disruption (Carbery *et al.,* 2010; Geurts *et al.,* 2009; Sung *et al.,* 2013; Tesson *et al.,* 2011). When a single stranded DNA (ssDNA) or a plasmid with homology flanking the double strand break site (DSB) is provided, a defined modification can be introduced into the genome by homologous recombination (HR) (Brown *et al.,* 2013; Cui *et al.,* 2011; Meyer *et al.,* 2010).

Recently, using the RNA-guided clustered regularly interspaced short palindromic repeat (CRISPR) / CRISPR-associated (Cas) nuclease system (Hsu *et al.,* 2014), methods for generation of mice carrying mutations in single and multiple genes, as well as mice carrying reporter and conditional alleles, in one step (Wang *et al.,* 2013; Yang *et al.,* 2013), have been established.

Using a similar approach, genetically modified animals have been generated from various species (Chang *et al.,* 2013; Hai *et al.,* 2014; Hwang *et al.,* 2013; Li *et al.,* 2013a; Li *et al.,* 2013b; Niu *et al.,* 2014). Similar to the traditional approach generating transgenic mice, these studies all employ microinjection to deliver nuclease components into the one-cell zygote.

The methodology described herein uses electroporation to deliver biological / genetic materials, such as the CRISPR/Cas system, to the mammalian (e.g., mouse) zygotes, and is successful to generate live transgenic animals (e.g., mice) carrying targeted gene modifications.

### Example 1 Electroporation of Mouse Zygotes and Presumptive Zygotes with Reporter-Encoding Polynucleotides

This experiment describes the result of electroporating mouse zygotes or presumptive zygotes ("zygotes" for short) with reporter-gene encoding plasmids.

The pMAXGFP vector (Lonza, USA), which carries the CMV promoter and the SV40 polyadenylation signal supporting a ubiquitous expression, was chosen for this experiment. The B6D2F2 mouse embryos were collected and treated for 5 seconds in Acidic Tyrode's solution (AT) (P/N T1788, Sigma Aldrich), washed twice in KOSMaa/BSA (P/N Zeks-050, Zenith Biotech), and placed into 25 µL of Opti-MEM (P/N 31985, Life Technologies). The embryos in 25 µL were then mixed with an equal volume (25 µL) of pMAXGFP at 80 ng/µL in TE buffer (10 mM Tris, 0.1 mM EDTA, pH 7.5) to arrive at a final DNA concentration of about 40 ng/µL, and the mixture was loaded into a 1-mm electroporation cuvette, and electroporated using the settings of: 30 volts, pulse duration 1 ms, two pulses, pulse interval 100 ms (ECM830, BTX).

After electroporation, the embryos were recovered with 100 µL of pre-equilibrated KOSMaa/BSA, and were subsequently cultured for 3.5 days in a tissue culture incubator (37°C, 5% CO₂). After 3.5 days in culture, the embryos were observed under a fluorescent microscope for GFP expression. Fluorescence was not observed among the 3 embryos examined (Experiment 1).

Since the zona pellucida layer may present a physical barrier for plasmid entry into the zygotes, in Experiments 2 and 3, Applicant sought to weaken or break the zona pellucida layer by poking a hole on the zona, by removing the zona, or by treating the embryos in AT for 10 seconds, while at the same time varying the concentrations of pMAXGFP from 20 ng/µL, to 40 ng/µL, to 100 ng/µL. Again, no fluorescence among the embryos examined was observed.

Other reporter systems, including an oligonucleotide labeled with 6-fluorescein amidite (6-FAM) (40, 100, and 500 ng/µL), Turbo GFP mRNA (40 ng/µL), eGFP mRNA (25 ng/µL) or mCherry mRNA (40 and 100 ng/µL) and did not observe fluorescence among the embryos examined (Experiments 4, 5, and 6). Only one embryo with GFP signal was observed in one case, but the embryo was dead or developmentally retarded.

### Example 2 Electroporation of Mouse Zygotes and Presumptive Zygotes with CRISPR/Cas System

This experiment demonstrates the delivery of CRISPR/Cas system, using the methods of the invention, to mouse zygotes or presumptive zygotes ("zygotes" for short) for CRISPR/Cas-mediated targeted gene disruption.

For this purpose, Tetl exon 4 and Tet2 exon 3 were chosen as targets, using the guide RNAs described previously (Wang *et al.,* 2013). The convenience of the Tetl and Tet2 systems is that the Sacl (Tetl) or the EcoRV (Tet2) restriction sites overlap the PAM (protospacer adjacent motif) proximal sequences. As such, Restriction Fragment Length Polymorphism (RFLP) analysis can be used to detect mutant alleles, using PCR products amplified from embryos that encompass the target sites (Wang *et al.,* 2013).

In Experiment 6, mouse B6D2F2 zygotes were first treated with AT for 10 seconds, mixed with Cas9 mRNA/Tet1 sgRNA at 40/20 ng/µL or 100/50 ng/µL, and electroporated as described. The embryos were then cultured for 3.5 days until they developed into blastocysts. The embryos were then harvested for whole genome amplification and PCR analysis.

PCR products encompassing the target site were analyzed by RFLP using the Sacl restriction enzyme. When the mixture of Cas9 mRNA (40 ng/µL) and Tetl sgRNA (20 ng/µL) was used, no mutation was detected among the embryos developed from the electroporated zygotes. When the mixture of Cas9 mRNA (100 ng/µL) and Tetl sgRNA (50 ng/µL) was used, 3 out of 16 AT-treated embryos and 1 out of 16 embryos without AT treatment were found to carry mutant alleles at the Tetl locus, based on RFLP analysis (FIG. 1A). The mutations were further confirmed by Sanger sequencing (FIG. 1A).

Next, in Experiment 7, an even higher concentration of Cas9 mRNA (400 ng/µL) + Tet2 sgRNA (200 ng/µL) were electroporated into mouse zygotes, and 4 out of 9 AT-treated embryos were found to carry biallelic mutations at the Tet2 locus (FIG. 1B).

Therefore, both Tetl and Tet2 genes can be efficiently targeted in mouse zygotes using electroporation-delivered CRISPR/Cas system (e.g., Cas9 mRNA + sgRNA).

Meanwhile, electroporation of CRISPR/Cas-encoding plasmids (as an alternative to the delivery of CRISPR reagents in the form of Cas mRNA and guide RNA) was attempted using different plasmid concentrations, namely 200 ng/µL, 400 ng/µL, and 800 ng/µL plasmid DNA. In the 200 ng/µL group, one mutant embryo out of 12 total screened was identified. Therefore, electroporation of DNA into zygotes can also be achieved, although in the case of CRISPR/Cas system, the mRNA/guide RNA combination appeared to be more efficient than DNA.

In this experiment and the other experiments using the CRISPR/Cas system, Cas9 mRNA and sgRNA were produced using the following procedure.

Briefly, the px330 plasmid carrying the wild type Cas9 gene (Cong et al., 2013) served as the DNA template for amplification of the Cas9 coding sequence in a polymerase chain reaction (PCR). The T7 promoter sequence was added to the forward primer and paired with the reverse primer flanking the coding sequence of the Cas9 wild type gene. PCR product was purified using the Qiagen column (Qiagen), and *in vitro* transcription (IVT) was performed using the mMESSAGE mMACHINE T7 ULTRA Transcription kit (Life Technologies). For sgRNA synthesis, the T7 promoter sequence was added to sgRNA template/forward primer and the IVT template was generated by PCR amplification. The T7-sgRNA PCR product was column purified and used as the template for IVT using MEGAshortscript T7 kit (Life Technologies). Both the Cas9 mRNA and the sgRNAs were purified using MEGAclear kit (Life Technologies) and eluted in elution buffer provided with the kit. Aliquots from an IVT reaction were separated on agarose gel to assess quality from a reaction.

Surveyor assay was performed as described (Guschin et al., 2010). Genomic DNA from mice or embryos was extracted and PCR performed using gene-specific primers under the following conditions: 95°C for 5 min; 35 × (95°C for 30 s, 60°C for 30 s, 68°C for 40 s); 68°C for 2 min; hold at 4°C. PCR products were then denatured, annealed, treated with Surveyor nuclease (Transgenomic). The products were then separated on a 10% acrylamide Criterion TBE gel (BioRad), stained with ethidium bromide and visualized. For RFLP analysis, 10 µl of Tet1 or Tet2 PCR products were digested with Sacl (Tetl) or EcoRV (Tet2) and separated on a GelRed (Biotium)-stained agarose gel (2%). For sequencing, PCR products were sequenced directly or cloned into the Topo Cloning Kit (Invitrogen), and individual clones sequenced by Sanger sequencing.

### Example 3 Development of Electroporated Mouse Zygotes

*In vitro* culture and analysis of zygotes is an important approach, based on which a large number of parameters related to gene editing technologies can be tested and analyzed, with a quick turnaround time and preferably a lower operating cost. However, when conventional *in vitro* culture system was used to culture electroporated zygotes, subsequent embryonic development seemed to be retarded or aborted. Often, after electroporation of Cas9 mRNA/sgRNA at varied concentration ranges of, for example, 50/25 ng/µLto 1000/500 ng/µL, embryos progressed to different stages of development at the end of the 3.5-day culture period, including those of 1-cell, 2-cell, or 4-cell stages, morula stage, and occasionally blastocyst stage (Experiment 8). In contrast, control embryos that had not been electroporated reached the blastocyst stage at the end of the same time period, whether the control embryos had been pre-treated with AT or not. In some experiments (Experiments 10 and 11), embryos among all the groups in an experiment failed to reach blastocyst stage.

Such initial failures in culturing electroporated zygotes appear to suggest that zygotes may not survive the electroporation process and continue to develop into live-born animal, regardless of whether the desired genetic modification occurs in the electroporated zygotes. The realization that AT treatment could damage the zygotes excessively to retard embryonic development, and that the reagents delivered, such as the Cas9 mRNA and the guide RNA, could be toxic to the zygotes and their subsequent development, further casts doubts as to using electroporation as a viable means to permanently introduce germline transmittable genetic modifications in the animal.

In attempting to resolve the poor *in vitro* development issue of the electroporated zygotes, Applicant surprisingly identified a solution by changing the *in vitro* culturing condition (Table 1), more specifically, by washing away any potentially harmful substance in the electroporation medium, which typically comprise a polynucleotide buffer *(e.g.,* TE buffer) and reduced-serum medium (e.g., OPTI-MEM^{®} medium). The dramatic effects of this, on survival and development of the electroporated zygotes, are demonstrated in the experiments described here.

In particular, B6D2F1/J donor female mice were superovulated to maximize embryo yield. Each donor female received 5 IU (*i.p.* injection) of Pregnant Mare Serum Gonadotrophin (PMSG), and, about 47 hours later, followed by 5 IU (*i.p.* injection) of human chorionic gonadotrophin (hCG).

Immediately post administration of hCG, the female was mated 1:1 with a B6D2F1/J stud male. About 24 hours later, the female was checked for the presence of a copulation plug. Females displaying a copulation plug were euthanized and their oviducts were excised and placed into M2 media. The oviducts were then placed in M2 media with hyaluronidase added at a concentration of about 0.3 mg/mL. The oocyte clutch was removed by lysing the ampulla and allowing to incubate in the M2 medium containing hyaluronidase, until the cumulus cells fell off. Zygotes were collected and passed through two washes of fresh M2 media before being placed in microdrops of RCVL media that had been equilibrated under oil for 24 hours in a COOK MINC benchtop incubator.

Acidic Tyrode's solution was thawed, and 100 µL drops were placed in a petri dish, with 1 drop for every 50 zygotes to be treated. Zygotes identified for EP (electroporation) were removed from the RCVL media, and placed in a (pre-warmed) 100 µL drop of M2 media. In groups of 50, the zygotes were placed in the Acidic Tyrode's solution for 10 seconds, and then removed and washed through three 100 µL drops of pre-warmed M2 media. The treated zygotes were then placed into 5-10 µL drops of OPTI-MEM^{®} medium in preparation for electroporation. The number of zygotes per OPTI-MEM^{®} medium drop and the number of drops varied with the parameters of the experiment being performed.

For both Experiments 14 and 15, CRISPR mixture contains Cas9 mRNA/Tet2 sgRNA at 200/100, 400/200, and 600/300 ng/µL were used. In addition, for Experiment 15, a donor oligonucleotide carrying mutation for converting the EcoRV site (GATATC) into a EcoRI site (GAATTC) was also provided at 200, 400, or 200 ng/µL. The mixture was brought up to 10 µL per experiment group in TE buffer (10 mM Tris, 0.1 mM EDTA, pH7.5) and added to embryos in 10 µL OPTI-MEM^{®}. The total content of 20 µL was then loaded into a 1-mm cuvette and electroporation delivered (30 volts, pulse duration 1 ms, 2 pulses, pulse interval 100-1000 ms).

Upon completion of electroporation, if it is desirable to wash the electroporated gametes or preimplantation embryos prior to the next steps *(e.g., in vitro* culturing and/or transferring to a pseudopregnant female), the embryos were recovered from the cuvette in 100 µL of pre-warmed KSOMaa/BSA media and serially passed through three 100 µL drops of pre-warmed M2 media to wash off any remaining electroporation solution (1:1 of TE buffer and OPTI-MEM^{®} medium). The zygotes were then assessed for viability. Those judged to be healthy were transferred into a cryovial containing 950 µL of pre-warmed M2 media for transport to the specific pathogen free (SPF) surgical suite.

In the SPF facility, the zygotes were removed from the cryovial using a p1000 pipette and placed into culture drops of RCVL media that had been equilibrated under oil for 24 hours in a COOK MINC benchtop incubator. At the time of transfer, the embryos were removed from the RCVL microdrop and placed in a pre-warmed 100 µL drop of M2 media and transferred into a CBYB6F1/J pseudo pregnant female in accordance to standard protocol, such as Jackson Laboratories LAH (laboratory animal health) routine procedure 94-09.

A subset of the mice were analyzed upon births. For these, tail biopsy samples were taken when the mice were born and analyzed by RFLP with EcoRV digestion (FIGs. 2A and 2B). PCR product from two mice out of 13 screened from the group of Cas9 mRNA 400 ng/µLand sgRNA 200 ng/µL was resistant (founder 85C3) or partially resistant to (85C10) to EcoRV digestion (FIG. 2, Panel A).

When Cas9 mRNA and sgRNA were used a higher concentration of 600 ng/µL and 300 ng/µL, respectively, five mice were positive as analyzed by the RFLP assay (founders 86C3, 86C5, 86C7, 86C8 and 86C9), among which two (86C3 and 86C9) were completely resistant while 3 (86C5, 86C7 and 86C8) were partially resistant to EcoRV digestion. Sanger sequencing of the PCR products confirmed presence of the mutant alleles for 6 out of the 7 mice (FIG. 2C, 85C3, 85C10, 86C3, 86C7, 86C8 and 86C9), while one may carry mutant alleles of low abundance (86C5). Furthermore, when the PCR products from founders 85C3, 86C3 and 86C9 were cloned into the pCR2.1-Topo vector and individual clones sequenced, INDEL mutations were detected, as expected, precisely located at the PAM proximal region of the Tet2 target site (FIG. 2D).

Remaining mice from these two experiments were analyzed when they were one week old. Tail biopsy samples were taken and analyzed as previously described by RFLP, and the results confirmed by Sanger sequencing of PCR products amplified from these mice. As summarized in Table 1, it was observed a concentration dependent improvement in founder efficiency between these two experiments. When Cas9 mRNA was used at 200 ng/µLand sgRNA 100 ng/µL, 1 founder was identified among the 34 screened (3.2%). When the concentration was increased to Cas9 mRNA 400 ng/µLand sgRNA 200 ng/µL, founder efficiency increased to 48.3% (14 founder mice among the 29 screened). When the concentration was further increased to Cas9 mRNA 600 ng/µL and sgRNA 300 ng/µL, efficiency was 45.5% (15 founder mice among the 33 screened). Remarkably, for Experiment 15, 100% founder efficiency was achieved, with all 11 mice screened found to carry mutant alleles (FIG. 3).

CRISPR-mediated HDR was also tested in Experiment 15, in which donor oligonucleotide was incorporated in the experimental design.

Specifically, zygotes were electroporated and mice were born and genotyped. A PCR product of 466 bps was amplified, which amplification product encompasses the target site, exposed to EcoRV digestion to detect alleles carrying NHEJ mutations and to EcoRI digestion to detect the HDR allele. Although successful HDR alleles were not detected among other experimental groups, two founder mice carrying alleles that were cleaved by EcoRI (Figure 3, Panel A, 86EP11 and 86EP14) were detected, and they were obtained from the group that was electroporated with Cas9 mRNA at 400 ng/µL, sgRNA targeting the 3' UTR of the Tet2 locus at 200 ng/µL, and ssDNA donor at 400 ng/µL. Among this group, all 11 mice carry alleles that are resistant to EcoRV digestion. Founders 86EP11 to 86EP15 do not seem to carry a detectable level of alleles that could be cleaved by EcoRV, suggesting presence of NHEJ mutations from all 11 founder mice. When the 11 samples were exposed to EcoR1, some of the alleles from samples 86EP11 and 86EP14 were shown to be partially digested by EcoRI, suggesting presence of the HDR alleles from these two samples.

PCR products were then cloned from founders 86EP11 and 86EP14 into the pCR2.1-TOPO vector, and individual clones were sequenced. As shown in FIG. 3, Panel B, sample 86EP11-4 and 86EP14-4 carry the EcoRI sites, suggesting successful incorporation of this site from donor ssDNA mediated by CRISPR/Cas.

A variety of different electroporation set up parameters were further tested in a series of experiments, and their results were summarized below:
1. **20V, 2 pulses, 100 ms interval, 0 washes**
   a. Cas9 mRNA 200 ng/uL + Tet2 sgRNA 100 ng/µL
   b. Results: 12% reached blastocyst stage (2/17), no gene targeting observed
2. **20V, 2 pulses, 100 ms interval, 3 washes**
   a. Cas9/Tet 2: 500/250, 400/200, 200/100 ng/µL
   b. Results: 91% blastocyst on average (50/55)
3. **20V, 2 pulses, 1 s interval, 3 washes**
   a. Cas9/Tet2: 800, 600, 400, 300, 200, 100 ng/µL
   b. Results (batch 1) at 200 ng/µL: 100% blast (16/16), 14.3% targeting (1/7)
   c. Results (batch 2) at 200, 300, 400 ng/µL: 95.8% blasts (46/48), results pending
   d. Results (batch 3): multiple washes, embryos transferred to pseudopregnant female mice, births pending
**4. 20V, 3 pulses, 1 s interval, 1 wash**
   a. Cas9/Tet2: 400, 200 ng/µL
   b. Results (batch 1) at 200 ng/µL: 82.3% blasts (14/17), no targeting
   c. Results (batch 2) at 400 and 200 ng/µL: 93.8% blasts (30/32), results pending
**5. 20V, 3 pulses, 1 s interval, 3 washes**
   a. Cas9/Tet2: 200 ng/µL
   b. Results: 88.9% blasts (16/18), no targeting
6. **30V, 2 pulses, 1 s interval, 3 washes**
   a. Cas9/Tet2: 400, 300, 200 ng/µL
   b. Results at 200 ng/µL: 94% blast (15/18), no targeting
   c. Results at 300 ng/µL: 84% blast (16/19), 16% targeting (3/19), no targeting at 400 ng/µL

In summary, the results suggest that successful genetic modification using electroporated CRISPR/Cas system depends on the concentration of the CRISPR/Cas reagents used. In an electroporation experiment, as compared to the traditional microinjection experiments, reagent concentration stops being a limiting factor, and it was found that the use of higher concentration reagents (e.g., above 400 ng/µL for Cas9 mRNA and 200 ng/µL for sgRNA) generates gene edited mice.

The data also suggests that weakening Zona Pellucida, e.g., by AT treatment, may be beneficial for zygote electroporation. However, prolonged treatment should be avoided.

Lastly but not the least, CRISPR-mediated gene targeting using the subject methods could be successfully conducted and with certain variations in parameters in experimental setup. To ensure successful development of the electroporated zygotes, it may be required to rinse once or multiple times *(e.g.,* 2, 3, 4 times) the electroporated zygotes with a physiological solution or media optimal for embryo development, upon completion of electroporation. For example, the electroporated zygotes can be serially passed through (100 µL) drops of pre-warmed M2 media to wash off any remaining electroporation solution (e.g., 1:1 of TE buffer and OPTI-MEM^{®}) before the zygotes are placed into culture or transferred to the uterine environment.

**Table 1 Results Summary for Experiments 14 and 15**

| BTX ECM830 Condition | | | | | | # Embryos | | | | | | |
|---|---|---|---|---|---|---|---|---|---|---|---|---|
| Experiment | Well ID | AT (s) | Voltage (v) | # Pulses | Pulse Interval (ms) | Pulse Duration (ms) | at EP | post EP | CRISPR Reagents | Conc. (ng/ul) | NHEJ Mutant/Total Mice | Percentage (%) |
| 14 | A1 | 10 | 27 | 2 | 125 | 1.5 | 20 | 17 | Cas9/Tet2 sgRNA | 200/100 | 0/11 | 0 |
| | B1 | 10 | 27 | 2 | 128 | 1.5 | 20 | 18 | Cas9/Tet2 sgRNA | 400/200 | 1/10 | 10 |
| | C1 | 10 | 27 | 2 | 128 | 1.5 | 20 | 18 | Cas9/Tet2 sgRNA | 600/300 | 4/6 | 67 |
| | D1 | 10 | 27 | 2 | 125 | 1.5 | 37 | 35 | Cas9/Tet2 sgRNA | 200/100 | 1/10 | 10 |
| | E1 | 10 | 27 | 2 | 128 | 1.5 | 37 | 34 | Cas9/Tet2 sgRNA | 400/200 | 2/8 | 29 |
| | F1 | 10 | 27 | 2 | 128 | 1.5 | 37 | 26 | Cas9/Tet2 sgRNA | 600/300 | 4/8 | 50 |
| | G1 | 10 | 27 | 2 | 128 | 1.5 | 20 | 17 | control | | 0/8 | 0 |
| | H1 | no AT | no EP | | | | 12 | 12 | control | | 0/3 | 0 |
| 15 | A1 | 10 | 27 | 2 | 128 | 1.5 | 20 | 18 | Cas9/Tet2 sgRNA/donor Cas9/Tet2 | 200/100/200 | 0/10 | 0 |
| | B1 | 10 | 27 | 2 | 130 | 1.5 | 20 | 20 | sgRNA/donor Cas9/Tet2 | 400/200/400 | 11/11 | 100 |
| | C1 | 10 | 27 | 2 | 130 | 1.5 | 20 | 19 | sgRNA/donor Cas9/Tet2 | 600/300/200 | 4/11 | 36 |
| | D1 | 10 | 27 | 2 | 128 | 1.5 | 40 | 36 | sgRNA/donor Cas9/Tet2 | 200/100/200 | 0/3 (no live | 0 |
| | E1 | 10 | 27 | 2 | 130 | 1.5 | 40 | 35 | sgRNA/donor Cas9/Tet2 | 400/200/400 | births) | |
| | F1 | 10 | 27 | 2 | 128 | 1.5 | 40 | 32 | sgRNA/donor | 600/300/200 | 3/8 | 38 |
| | G1 | 10 | 27 | 2 | 130 | 1.5 | 20 | 19 | control | | 0/12 | 0 |
| | H1 | no AT | no EP | | | | 14 | 14 | control | | 0/10 | 0 |

A detailed illustrative (but non-limiting) embodiment of the method of the invention is provided herein below:
1. Aliquots of 100 µL KSOMaa Evolve (P/N Zeks-050, Zenith Biotech), supplemented with BSA (P/N A2153, Sigma-Aldrich) at 1 mg/mL, were deposited onto a 96-well flat bottom tissue culture plate and equilibrated in HeraCell incubator at 37°C/5% CO₂, prior to receipt of one cell zygotes.
2. B6D2F1 female mice were injected intraperitoneally (*i.p.*) with 5 international units (IU) of pregnant mare's serum gonadotropin (PMSG, P/N HOR-272, ProSpec, Rehovot, Israel), and were followed 48 hours later with an *i.p.* injection of human chorionic gonadotropin (hCG, P/N HOR-250, ProSpec, Rehovot, Israel). The female mice were then mated with B6D2F1 male mice. Those that were positive for a copulation plug were euthanized by cervical dislocation (CD). Fertilized embryos were flushed from the excised reproductive tracts.
3. One cell zygotes from B6D2F2 mice were collected, treated for 10 seconds in Acidic Tyrode's Solution (e.g., Sigma-Aldrich, Cat. No. T1788; or equivalent such as EMBRYOMAX^{®} P/N MR-004-D, EMD Millipore), washed twice with KSOMaa Evolve, and placed in 10 µL drops of prewarmed OPTI-MEM^{®} medium (P/N 31985, Gibco) in a tissue culture dish or plate (35 mm, P60, 96 well).
4. "Biological material" (for CRISPR, it consists essentially of Cas9 mRNA, sgRNA, and optionally donor oligonucleotide; or DNA encoding the same as an alternative) was reconstituted into a 10 µL volume with 10 mM Tris, pH 7.5, and 0.1 mM EDTA, and added to the 10 µL drop of zygotes in OPTI-MEM^{®} medium.
5. Zygotes immersed in the "biological material" were removed and deposited into the chamber of the 2-mm cuvette for BTX Model 610 (P/N 450124, Harvard Apparatus). Tap the cuvette to ensure polynucleotide/zygote solution or suspension move to between the two conductors of the cuvette, with no air bubble trapped in the mixture.
6. Place cuvette into the BTX cuvette chamber (ECM 830) and electroporate using the following settings (or variations thereof, such as those disclosed herein):
   a. Voltage: 30V
   b. Pulse Interval: 125-130 ms
   c. Pulse Duration: 1 ms
   d. # Pulses: 2
7. Using supplied plastic pipette from cuvette package, remove 80-100 µL of prewarmed media (e.g., KSOMaa/BSA media) from respective well of a 96-well plate and gently add the media to the cuvette. Remove all contents from the cuvette and expel the mixture to the well.
8. Serially passing the electroporated zygotes through two-three 100 µL drops of pre-warmed M2 media to wash off any remaining electroporation solution.
9. Immediately place the 96-well plate carrying the washed zygotes back into the incubator.
10. Embryos were cultured at 37°C/5% CO₂ for 3.5 days to reach blastocyst stage of development and then harvested for analysis.
11. Genomes of the embryos were amplified using the GENOMEPLEX^{®} Single Cell Whole Genome Amplification Kit (P/N GA4, Sigma-Aldrich). Amplified genome was then used as template in a PCR reaction (ACCUPRIME^{™} Taq DNA Polymerase System, P/N 12339-016, Life Technologies; or PrimeStar GXL, P/N R050B, Clontech) with a primer pair encompassing the target site. PCR products were cleaned and nucleotide sequences analyzed by Sanger sequencing.
12. Cas9 mRNA, sgRNA and the optional DNA donor were synthesized, and injection mixture prepared as described previously (Wang *et al.,* 2013; Yang *et al.,* 2013).

### References

Bogdanove, A.J., and Voytas, D.F., "TAL effectors: customizable proteins for DNA targeting," Science, 333:1843-1846 (2011).

Brown, A.J., Fisher, D.A., Kouranova, E., McCoy, A., Forbes, K., Wu, Y., Henry, R., Ji, D., Chambers, A., Warren, J., et al., "Whole-rat conditional gene knockout via genome editing," Nat. Methods, 10:638-640 (2013).

Capecchi, M.R., "Gene targeting in mice: functional analysis of the mammalian genome for the twenty-first century," Nat. Rev. Genet., 6:507-512 (2005).

Carbery, I.D., Ji, D., Harrington, A., Brown, V., Weinstein, E.J., Liaw, L., and Cui, X., "Targeted genome modification in mice using zinc-finger nucleases," Genetics, 186:451-459 (2010).

Chang, N., Sun, C., Gao, L., Zhu, D., Xu, X., Zhu, X., Xiong, J.W., and Xi, J.J., "Genome editing with RNA-guided Cas9 nuclease in Zebrafish embryos," Cell Res., 23:465-472 (2013).

Cui, X., Ji, D., Fisher, D.A., Wu, Y., Briner, D.M., and Weinstein, E.J., "Targeted integration in rat and mouse embryos with zinc-finger nucleases," Nat. Biotechnol., 29:64-67 (2011).

Geurts, A.M., Cost, G.J., Freyvert, Y., Zeitler, B., Miller, J.C., Choi, V.M., Jenkins, S.S., Wood, A., Cui, X., Meng, X., et al. "Knockout rats via embryo microinjection of zinc-finger nucleases," Science, 325:433 (2009).

Hai, T., Teng, F., Guo, R., Li, W., and Zhou, Q., "One-step generation of knockout pigs by zygote injection of CRISPR/Cas system," Cell Res., 24:372-375 (2014).

Hsu, P.D., Lander, E.S., and Zhang, F., "Development and Applications of CRISPR-Cas9 for Genome Engineering," Cell, 157:1262-1278 (2014).

Hwang, W.Y., Fu, Y., Reyon, D., Maeder, M.L., Tsai, S.Q., Sander, J.D., Peterson, R.T., Yeh, J.R., and Joung, J.K., "Efficient genome editing in zebrafish using a CRISPR-Cas system," Nat. Biotechnol., 31:227-229 (2013).

Li, D., Qiu, Z., Shao, Y., Chen, Y., Guan, Y., Liu, M., Li, Y., Gao, N., Wang, L., Lu, X., et al., "Heritable gene targeting in the mouse and rat using a CRISPR-Cas system," Nat. Biotechnol., 31:681-683 (2013a).

Li, W., Teng, F., Li, T., and Zhou, Q., "Simultaneous generation and germline transmission of multiple gene mutations in rat using CRISPR-Cas systems," Nat. Biotechnol., 31:684-686 (2013b).

Meyer, M., de Angelis, M.H., Wurst, W., and Kuhn, R., "Gene targeting by homologous recombination in mouse zygotes mediated by zinc-finger nucleases," Proc. Natl. Acad. Sci. USA, 107:15022-15026 (2010).

Niu, Y., Shen, B., Cui, Y., Chen, Y., Wang, J., Wang, L., Kang, Y., Zhao, X., Si, W., Li, W., et al., "Generation of Gene-Modified Cynomolgus Monkey via Cas9/RNA-Mediated Gene Targeting in One-Cell Embryos," Cell, 156:836-843 (2014).

Sung, Y.H., Baek, I.J., Kim, D.H., Jeon, J., Lee, J., Lee, K., Jeong, D., Kim, J.S., and Lee, H.W., "Knockout mice created by TALEN-mediated gene targeting," Nat. Biotechnol., 31:23-24 (2013).

Tesson, L., Usal, C., Menoret, S., Leung, E., Niles, B.J., Remy, S., Santiago, Y., Vincent, A.I., Meng, X., Zhang, L., et al., "Knockout rats generated by embryo microinjection of TALENs," Nat. Biotechnol., 29:695-696 (2011).

Urnov, F.D., Rebar, E.J., Holmes, M.C., Zhang, H.S., and Gregory, P.D., "Genome editing with engineered zinc finger nucleases," Nat. Rev. Genet., 11:636-646 (2010).

Wang, H., Yang, H., Shivalila, C.S., Dawlaty, M.M., Cheng, A.W., Zhang, F., and Jaenisch, R., "One-step generation of mice carrying mutations in multiple genes by CRISPR/Casmediated genome engineering," Cell, 153:910-918 (2013).

Yang, H., Wang, H., Shivalila, C.S., Cheng, A.W., Shi, L., and Jaenisch, R., "One-step generation of mice carrying reporter and conditional alleles by CRISPR/Cas-mediated genome engineering," Cell, 154:1370-1379 (2013).

## Claims

1. A method of generating a genetically modified mouse, the method comprising:
(a) combining a preimplantation stage mouse embryo with an electroporation medium that comprises a guide RNA (gRNA) and at least 200 ng/µL messenger RNA encoding a Cas9 protein;
(b) electroporating the preimplantation stage mouse embryo in the electroporation medium to produce an electroporated mouse embryo comprising the gRNA and the mRNA encoding a Cas9 protein; and
(c) washing the electroporated mouse embryo of (b) in a physiological solution or medium; and
(d) transferring the electroporated mouse embryo of (c) into a pseudopregnant female mouse, wherein the electroporated mouse embryo develops into a live-born mouse comprising a genetic modification in the genomic target sequence, and wherein the live-born mouse represents an animal model corresponding to a human genetic disease or disorder.

2. The method of claim 1, wherein the concentration ratio for the mRNA encoding the Cas9 protein and the gRNA is at least about 1:1, at least about 1.5:1, at least about 2:1, at least about 2.5:1, at least about 3:1 or higher.

3. The method of claim 2, wherein the concentration of mRNA encoding the Cas9 protein is at least 300 ng/µL, at least 400 ng/µL, at least 500 ng/µL, at least 600 ng/µL, at least 800 ng/µL, at least 1000 ng/µL or higher

4. The method of any one of claims 1-3, wherein the solution further comprises a donor polynucleotide, wherein the donor polynucleotide is a linear or circular double stranded DNA molecule.

5. The method of any one of claims 1-4, wherein 2, 3, 5, 10, 20, 30, 40, 50, 100, 125, 150, 200, 250, 300 or more preimplantation stage embryos are simultaneously electroporated.

6. The method of claim 5, wherein at least about 50%, 60%, 70%, 80%, 85%, 90%, 95% or more of the electroporated preimplantation stage embryos develop into live-born transgenic mice.

7. The method of any one of claims 1-6, wherein the preimplantation stage embryo is pre-treated to weaken Zona Pellucida prior to electroporation.

8. The method of claim 7, wherein the preimplantation stage embryo is pre-treated in Acidic Tyrode's solution (AT) or equivalent.

9. The method of any one of claims 1-8, wherein electroporation is carried out in a 1-mm electroporation cuvette, using the settings of: 20-40 volts, pulse duration 1-2 ms, 1-3 pulses, pulse interval 100-1000 ms.

10. The method of any one of claims 1-9, wherein the preimplantation stage embryo is a zygote.

11. The method of any one of claim s 1-10, wherein the preimplantation stage mouse embryo is an inbred preimplantation stage mouse embryo.

12. The method of claim 11, wherein the inbred preimplantation stage mouse embryo is from a recombinant inbred mouse strain selected from the group consisting of C3H, CBA, DBA, FVB, NOD, BALB/c, 129, and C57BL.

13. The method of any one of claims 1-12, further comprising genotyping the live-born mouse.

14. The method of any one of claims 1-13, wherein the human genetic disease or disorder is selected from cancers, heart diseases, hypertension, metabolic and hormonal disorders, diabetes, obesity, osteoporosis, glaucoma, skin pigmentation diseases, blindness, deafness, neurodegenerative disorders, psychiatric disturbances, and birth defects.

15. The method of claim 14, wherein, if the human genetic disease or disorder is
(a) a neurodegenerative disorder, it is Huntington's or Alzheimer's disease;
(b) a psychiatric disturbance, it is anxiety or depression; or
(c) a birth defect, it is cleft palate or anencephaly.

## Patentansprüche

1. Verfahren zur Erzeugung einer genetisch modifizierten Maus, wobei das Verfahren Folgendes umfasst:
(a) Kombinieren eines Mausembryos in der Präimplantationsphase mit einem Elektroporationsmedium, welches eine Guide RNA (gRNA) und mindestens 200 ng/µL Messenger-RNA umfasst, welche für ein Cas9-Protein codiert;
(b) Elektroporieren des Mausembryos in der Präimplantationsphase in dem Elektroporationsmedium zum Erzeugen eines elektromorierten Mausembryos, umfassend die gRNA und die für ein Cas9-Protein codierende mRNA; und
(c) Waschen des elektroporierten Mausembryos aus (b) in einer physiologischen Lösung oder einem physiologischen Medium; und
(d) Transferieren des elektroporierten Mausembryos aus (c) in eine pseudoschwangere weibliche Maus, wobei sich der elektroporierte Mausembryo zu einer lebendgeborenen Maus entwickelt, umfassend eine genetische Modifikation in der Genom-Zielsequenz, und wobei die lebendgeborene Maus ein Tiermodell darstellt, welches einer menschlichen genetischen Erkrankung oder Störung entspricht.

2. Verfahren nach Anspruch 1, wobei das Konzentrationsverhältnis zwischen der für das Cas9-Protein codierenden mRNA und der gRNA mindestens ungefähr 1:1, mindestens ungefähr 1,5:1, mindestens ungefähr 2:1, mindestens ungefähr 2,5:1, mindestens ungefähr 3:1 oder darüber beträgt.

3. Verfahren nach Anspruch 2, wobei die Konzentration an für das Cas9-Protein codierender mRNA mindestens 300 ng/µL, mindestens 400 ng/µL, mindestens 500 ng/µL, mindestens 600 ng/µL, mindestens 800 ng/µL, mindestens 1000 ng/µL oder darüber beträgt.

4. Verfahren nach einem der Ansprüche 1 bis 3, wobei die Lösung ferner ein Spender-Polynukleotid umfasst, wobei das Spender-Polynukleotid ein lineares oder kreisförmiges doppelsträngiges DNA-Molekül ist.

5. Verfahren nach einem der Ansprüche 1 bis 4, wobei 2, 3, 5, 10, 20, 30, 40, 50, 100, 125, 150, 200, 250, 300 oder mehr Embryos in der Präimplantationsphase gleichzeitig elektroporiert werden.

6. Verfahren nach Anspruch 5, wobei mindestens ungefähr 50 %,60 %,70 %, 80 %, 85 %, 90 %, 95 % oder mehr der elektropolierten Embryos in der Präimplantationsphase sich zu lebendgeborenen transgenen Mäusen entwickeln.

7. Verfahren nach einem der Ansprüche 1 bis 6, wobei der Embryo in der Präimplantationsphase vorbehandelt wird, um die Zona pellucida vor der Elektroporation zu schwächen.

8. Verfahren nach Anspruch 7, wobei der Embryo in der Präimplantationsphase in saurer Tyrodelösung (AT) oder gleichwertig vorbehandelt wird.

9. Verfahren nach einem der Ansprüche 1 bis 8, wobei die Elektroporation in einer 1-mm Elektroporationsküvette mit folgenden Einstellungen ausgeführt wird: 20-40 Volt, Pulsdauer 1-2 ms, 1-3 Pulse, Pulsintervall 100-1000 ms.

10. Verfahren nach einem der Ansprüche 1 bis 9, wobei der Embryo in der Präimplantationsphase eine Zygote ist.

11. Verfahren nach einem der Ansprüche 1 bis 10, wobei der Mausembryo in der Präimplantationsphase ein inzüchtiger Mausembryo in der Präimplantationsphase ist.

12. Verfahren nach Anspruch 11, wobei der inzüchtige Mausembryo in der Präimplantationsphase aus einem rekombinanten Inzuchtstamm der Maus stammt, gewählt aus der Gruppe, bestehend aus C3H, CBA, DBA, FVB, NOD, BALB/c, 129 und C57BL.

13. Verfahren nach einem der Ansprüche 1 bis 12, ferner umfassend Genotypisieren der lebendgeborenen Maus.

14. Verfahren nach einem der Ansprüche 1 bis 13, wobei die menschliche genetische Erkrankung oder Störung gewählt ist aus Krebserkrankungen, Herzerkrankungen, Hypertonie, Stoffwechsel- und hormonellen Störungen, Diabetes, Adipositas, Osteoporose, Glaukom, Hautpigmentierungserkrankungen, Blindheit, Taubheit, neurodegenerativen Störungen, psychiatrischen Störungen und Geburtsschäden.

15. Verfahren nach Anspruch 14, wobei, wenn die menschliche genetische Erkrankung oder Störung:
(a) eine neurodegenerative Störung ist, es sich um die Huntington'sche oder Alzheimer'sche Krankheit handelt;
(b) eine psychiatrische Störung ist, es sich um Angst oder Depression handelt; oder
(c) ein Geburtsschaden ist, es sich um eine Gaumenspalte oder eine Anenzephalie handelt.

## Revendications

1. Procédé de génération d'une souris génétiquement modifiée, le procédé comprenant :
(a) la combinaison d'un embryon de souris au stade préimplantatoire avec un milieu d'électroporation qui comprend un ARN guide (ARNg) et au moins 200 ng/µL d'ARN messager codant pour une protéine Cas9 ;
(b) l'électroporation de l'embryon de souris au stade préimplantatoire dans le milieu d'électroporation pour produire un embryon de souris électroporé qui comprend l'ARNg et l'ARNm codant pour une protéine Cas9 ; et
(c) le lavage de l'embryon de souris électroporé de (b) dans une solution ou un milieu physiologique ; et
(d) le transfert de l'embryon de souris électroporé de (c) dans une souris femelle en état de pseudogestation, dans lequel l'embryon de souris électroporé se développe en une souris née vivante qui comprend une modification génétique dans la séquence cible génomique, et dans lequel la souris née vivante représente un modèle animal qui correspond à une maladie ou un trouble génétique de l'être humain.

2. Procédé selon la revendication 1, dans lequel le rapport de concentration pour l'ARNm codant pour la protéine Cas9 et l'ARNg est d'au moins environ 1:1, d'au moins environ 1,5:1, d'au moins environ 2:1, d'au moins environ 2,5:1, d'au moins environ 3:1 ou plus.

3. Procédé selon la revendication 2, dans lequel la concentration de l'ARNm codant pour la protéine Cas9 est d'au moins 300 ng/µL, d'au moins 400 ng/µL, d'au moins 500 ng/µL, d'au moins 600 ng/µL, d'au moins 800 ng/µL, d'au moins 1000 ng/µL ou plus.

4. Procédé selon l'une quelconque des revendications 1 à 3, dans lequel la solution comprend en outre un polynucléotide donneur, dans lequel le polynucléotide donneur est une molécule d'ADN à brin double linéaire ou circulaire.

5. Procédé selon l'une quelconque des revendications 1 à 4, dans lequel 2, 3, 5, 10, 20, 30, 40, 50, 100, 125, 150, 200, 250, 300 embryons au stade préimplantatoire ou plus sont simultanément électroporés.

6. Procédé selon la revendication 5, dans lequel au moins environ 50 %,60 %, 70 %, 80 %, 85 %, 90 %, 95 % ou plus des embryons au stade préimplantatoire électroporés se développent en souris transgéniques nées vivantes.

7. Procédé selon l'une quelconque des revendications 1 à 6, dans lequel l'embryon au stade préimplantatoire est prétraité pour affaiblir la zone pellucide avant l'électroporation.

8. Procédé selon la revendication 7, dans lequel l'embryon au stade préimplantatoire est prétraité dans une solution de tyrode acide (AT) ou équivalent.

9. Procédé selon l'une quelconque des revendications 1 à 8, dans lequel l'électroporation est mise en œuvre dans une cuvette d'électroporation de 1 mm, en utilisant les réglages suivants : 20-40 Volts, durée des impulsions 1-2 ms, 1-3 impulsions, intervalle des impulsions 100-1000 ms.

10. Procédé selon l'une quelconque des revendications 1 à 9, dans lequel l'embryon au stade préimplantatoire est un zygote.

11. Procédé selon l'une quelconque des revendications 1 à 10, dans lequel l'embryon de souris au stade préimplantatoire est un embryon de souris au stade préimplantatoire congénique.

12. Procédé selon la revendication 11, dans lequel l'embryon de souris au stade préimplantatoire congénique provient d'une souche de souris congénique recombinante sélectionnée parmi le groupe constitué par C3H, CBA, DBA, FVB, NOD, BALB/c, 129 et C57BL.

13. Procédé selon l'une quelconque des revendications 1 à 12, comprenant en outre le génotypage de la souris née vivante.

14. Procédé selon l'une quelconque des revendications 1 à 13, dans lequel la maladie ou le trouble génétique de l'être humain est sélectionné(e) parmi les cancers, les maladies cardiaques, l'hypertension, les troubles métaboliques et hormonaux, le diabète, l'obésité, l'ostéoporose, le glaucome, les maladies de la pigmentation de la peau, la cécité, la surdité, les affections neurodégénératives, les syndromes d'ordre psychiatrique et les anomalies congénitales.

15. Procédé selon la revendication 14, dans lequel, si la maladie ou le trouble génétique de l'être humain est
(a) une affection neurodégénérative, il s'agit de la maladie de Huntington ou d'Alzheimer ;
(b) un syndrome d'ordre psychiatrique, il s'agit de l'anxiété ou de la dépression ; ou
(c) une anomalie congénitale, il s'agit de la fente palatine ou de l'anencéphalie.
